(19) Europäisches Patentamt
European Patent Office
Office européen des brevets

(11) EP 4 810 593 A1

(12) EUROPEAN PATENT APPLICATION
published in accordance with Art. 153(4) EPC

(43) Date of publication:
**23.09.2026 Bulletin 2026/39**

(21) Application number: **24891222.2**

(22) Date of filing: **30.10.2024**

(51) International Patent Classification (IPC):
***C11D 1/14*** *(2006.01)* ***C11D 1/68*** *(2006.01)*
***C11D 3/22*** *(2006.01)*

(52) Cooperative Patent Classification (CPC):
**A61K 8/46; A61K 8/60; A61Q 19/10; C11D 1/14; C11D 1/83; C11D 3/2003; C11D 3/22**

(86) International application number:
**PCT/JP2024/038697**

(87) International publication number:
**WO 2025/105181 (22.05.2025 Gazette 2025/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.11.2023 JP 2023193978**
**19.04.2024 JP 2024068568**

(71) Applicant: **Kao Corporation**
**Tokyo 103-8210 (JP)**

(72) Inventor: **YAKITA, Yukiko**
**Tokyo 103-8210 (JP)**

(74) Representative: **Hoffmann Eitle**
**Patent- und Rechtsanwälte PartmbB**
**Arabellastraße 30**
**81925 München (DE)**

(54) **CLEANING AGENT COMPOSITION**

(57) The present invention relates to a cleansing composition for cleansing a hard-surface article, an apparel product, or a body, which can exert excellent cleansing performance, without being influenced by the hardness or temperature of water for use in cleansing, while a glycolipid biosurfactant is used. Specifically, the present invention provides a cleansing composition including the following components (A) to (C): (A) a glycolipid biosurfactant, (B) an internal olefin sulfonate having 8 or more and 24 or less carbon atoms, in which a content of an internal olefin sulfonate having a sulfonic acid group at the 2-position is 40% by mass or less, and (C) water.

EP 4 810 593 A1

## Description

Field of the Invention

The present invention relates to a cleansing composition, particularly, a cleansing composition suitable for cleansing a hard-surface article, an apparel product, or a body.

Background of the Invention

Biosurfactants are surface active substances which are mainly produced by microorganisms and the like and have distinctive functionality, and are broadly classified into, for example, glycolipid type, fatty acid type, peptide type, and polymer type. Among them, the glycolipid biosurfactants have received attention in many fields in recent years because of their high availability in terms of productivity and functionality, and the like, and cleansing compositions have also been developed by combining the glycolipid biosurfactants with various surfactants.

For example, Patent Literature 1 discloses an aqueous cleansing agent containing a glycolipid biosurfactant, and a nonionic surfactant such as fatty alcohol polyglycol ether and an anionic surfactant such as fatty alcohol sulfate, and, for example, it is intended to clean a hard surface or a soft surface. Patent Literature 2 discloses a cleansing composition containing a glycolipid biosurfactant, sorbitan ester having a specific HLB value, and other surfactants, and it is intended to use, for example, a cleansing composition for hard surfaces or a cleansing composition for ships.

(Patent Literature 1) WO 2015/091250
(Patent Literature 2) U.S. Patent Application Publication No. 2020/0199492

Summary of the Invention

The present invention provides a cleansing composition comprising the following components (A) to (C):

(A) a glycolipid biosurfactant,
(B) an internal olefin sulfonate having 8 or more and 24 or less carbon atoms, wherein a content of an internal olefin sulfonate having a sulfonic acid group at the 2-position is 40% by mass or less, and
(C) water.

In techniques described in the above patent literatures, a surfactant which is used in combination with a glycolipid biosurfactant is influenced by the hardness or temperature of water for use in cleansing. Thus, it may result in deteriorating cleansing performance. In order to prevent such reduction in cleansing performance, the amount of an additive (e.g., a builder), a solvent, or the like used tends to be excessive. This might be responsible for impairing stability of a composition since resulting in the deposition of the surfactant, thickening due to liquid crystal phase formation, or the like. Furthermore, in these patent literatures, there is no focus on the increasing risk of deteriorating the low-temperature stability of a composition with an increase in the order of structure of the glycolipid type biosurfactant and an increase in the length of the hydrophobic chain, and there is still room for improvement. Moreover, some surfactants which are used in combination with the glycolipid biosurfactant may influence protein elution, and hence might have damage not only on the hand skin upon application of the cleansing composition but also on natural fibers such as wool or silk to be cleansed.

In recent years, high quality or handleability has been required with increase in characteristics required by consumers, and colorless transparent appearance has been preferred. Nonetheless, it found that the conventional techniques were unable to sufficiently address these demands.

Thus, the present invention relates to a cleansing composition which can exert excellent cleansing performance, without being influenced by the hardness or temperature of water for use in cleansing, while a glycolipid biosurfactant is used, and further, is excellent in fluidity and exhibits favorable appearance.

Further, the present invention relates to a cleansing composition which can effectively suppress protein elution and therefore effectively reduce not only damage on the hand skin upon application of the cleansing composition, but also damage on natural fibers such as wool or silk to be cleansed.

The present inventor conducted diligent studies. As a result of that, the inventor found that if an internal olefin sulfonate having a specific number of carbon atoms and having a specific amount of an abundance ratio of a sulfonic acid group at the 2-position is contained together with a glycolipid biosurfactant, the resulting cleansing composition addresses a wide range of conditions of water for use in cleansing and stably exerts excellent cleansing performance.

The cleansing composition of the present invention, when diluted upon cleansing, can stably exert excellent cleansing performance without being influenced by the hardness or temperature of the water used while avoiding the formation of unnecessary deposits. Furthermore, the cleansing composition of the present invention is capable of reducing

the amount of an additive (e.g., a chelating agent), a solvent, or the like used, and hence can achieve a highly useful cleansing composition with consideration for environmental load.

Moreover, in the cleansing composition of the present invention, individual components are uniformly dissolved, and it has moderate fluidity. Therefore, when a container is filled with the cleansing composition, the cleansing composition can be taken out of the container without varying a composition. Furthermore, because of its excellent colorless transparency, excellent cleansing performance can be effectively and pleasantly realized without being influenced by a usage manner.

Besides, the cleansing composition of the present invention can effectively suppress protein elution, and therefore effectively reduce not only damage on the hand skin upon application of the cleansing composition, but also damage on natural fibers such as wool or silk to be cleansed.

Detailed Description of the Invention

Hereinafter, the present invention will be described in detail.

In the present invention, the "stability" of a cleansing composition or a cleansing agent means that, for example, unnecessary deposition of a contained component, liquid crystal phase formation, and excessive thickening are prevented, and a state where individual components are uniformly dissolved (hereinafter, also referred to as "uniform solubility") is retained.

In the present invention, the "colorless transparency" which is exhibited by a cleansing composition or a cleansing agent means a state with high lightness and high transparency. Thus, the colorless transparency is meant to encompass not only a colorless state which exhibits favorable transparency, but also even a colored state which maintains lightness without practical problems and has transparency. Thus, "excellent colorless transparency" means that a cleansing composition or a cleansing agent exhibits excellent appearance and is applicable to a wider range.

Various subjects can be cleansed with the cleansing composition of the present invention. The cleansing composition of the present invention is particularly preferable as a composition for cleansing a hard-surface article, an apparel product, or a body, with which a hard-surface article, an apparel product, or a body is to be cleansed. Specifically, the cleansing composition of the present invention is suitable as a cleansing composition with which a hard-surface article, an apparel product, or a body is to be cleansed (hereinafter, also referred to as the "cleansing composition" of the present invention), and can be preferably used as a cleansing composition for a hard-surface article, a cleansing composition for an apparel product, or a cleansing composition for the body.

The cleansing composition of the present invention can also be used for the purpose of cleansing various dirt. Specific examples of the dirt include sebum, mud, beef tallow, foundations, lipsticks, food-derived pigments such as carotene, scum, and water scale.

The hard-surface article means an article with its surface formed from a resin, a plastic, a metal, glass, pottery, wood, marble, or a combination thereof.

The apparel product means an article produced using a fiber. Specific examples of the fiber include: protein fibers; resin fibers such as polyester fibers and polyvinylidene chloride fibers; hydrophobic fibers such as glass fibers, carbon fibers, and metal fibers (gold threads, silver threads, and steel fibers); and hydrophilic fibers such as cotton, linen, silk fibers, wool, rush, and straw.

The fiber is meant to exclude hair. The fiber further excludes fibers for use in products for wearing, modification, or the like for hair or the scalp. Specifically, the fiber also excludes even "fibers for headwear products" such as hair wigs, hair pieces, weavings, hair extensions, braid hair, hair accessories, and doll hair.

Specific examples of the apparel product produced using the fiber include fabrics such as woven fabrics, knitted fabrics, and nonwoven fabrics, and products such as T-shirts, dress shirts, blouses, knit, slacks, hats, towels, handkerchiefs, socks, underwear, tights, and masks produced using these fabrics.

The apparel product excludes headwear products.

The body means the arms, the legs, the back, the hands, the neck, the face, the breast, and the abdomen with their surfaces composed of the skin.

The body excludes the head with its surface composed of the scalp.

The cleansing composition of the present invention comprises a glycolipid biosurfactant as the component (A). Biosurfactants are broadly classified, from the structures of their hydrophilic groups, into glycolipid type, fatty acid type, peptide type, polymer type, and the like. In the present invention, a glycolipid biosurfactant composed of a glycan and a lipid is contained therein.

Specifically, the cleansing composition of the present invention comprises a specific internal olefin sulfonate as the component (B) mentioned later, together with the glycolipid biosurfactant as the component (A), whereby their characteristic structures or performance exert synergistic effects and can secure favorable stability of the composition by effectively preventing unnecessary deposits or the like, without being influenced by the hardness or temperature of water for use in cleansing. Thus, the cleansing composition of the present invention can exert excellent cleansing performance

without the need of containing an additive such as a chelating agent, or a solvent.

Specific examples of the component (A) include one or more selected from the group consisting of sophorolipid, rhamnolipid, trehalose lipid, and mannosyl alditol lipid. These components (A) may be protonated or may form a salt.

The sophorolipid has a structure where long-chain hydroxy fatty acid is bonded to sophorose, and can include a lactonic form (LSL) prepared through a cyclic ester bond formed by a carboxyl group of the long-chain hydroxy fatty acid and a hydroxy group of the sophorose, and an acidic form (ASL) prepared by hydrolysis thereof.

The lactonic form of sophorolipid is represented by the following formula (1), and the acidic form of sophorolipid is represented by the following formula (2):

$R^2O$, HO, HO, $R^1O$, O, HO, OH, $R^3$, $R^4$, O

··· (1)

$R^2O$, HO, HO, $R^1O$, HO, HO, OH, O, $R^3$, $R^4$, C, O, OH

··· (2)

wherein $R^1$, $R^2$, $R^3$, and $R^4$ are synonymous, and $R^1$ and $R^2$ each independently represent H or an acetyl group; $R^3$ represents a saturated or unsaturated hydrocarbon group having 1 or more and 9 or less carbon atoms; and $R^4$ represents a saturated or unsaturated hydrocarbon group having 1 or more and 19 or less carbon atoms.

For example, a launched product such as BioToLife(R) (manufactured by BASF SE) or REWOFERM SL ONE(R) (manufactured by Evonik Industries AG) can be used as the sophorolipid.

The rhamnolipid has a structure where long-chain hydroxy fatty acid is bonded to rhamnose, and is represented by the following formula (3):

... (3)

wherein m and n each independently represent an integer of 1 or 2; a represents an integer of 4 or larger and 10 or smaller; $R^5$ represents H or $CH_3(CH_2)_bCH{=}CHCO$-; b represents an integer of 4 or larger and 10 or smaller; and $R^6$ represents H or a cation.

For example, a launched product manufactured by Sigma-Aldrich Co., LLC can be used as the rhamnolipid.

The trehalose lipid is constituted by trehalose and a fatty acid or an acid and represented by the following formula (4):

... (4)

wherein $R^7$, $R^8$, and $R^9$ each independently represent a saturated or unsaturated hydrocarbon group having 5 or more and 13 or less carbon atoms.

The mannosyl alditol lipid has a structure where sugar alcohol is bonded to mannose. Examples thereof include mannosyl erythritol lipid (MEL), mannosyl mannitol lipid (MML), mannosyl sorbitol lipid (MSL), mannosyl arabitol lipid (MAraL), and mannosyl ribitol lipid (MRL). Among them, mannosyl erythritol lipid of the following formula (5) is preferred:

... (5)

wherein $R^{10}$ and $R^{11}$ each independently represent H or an acetyl group; and p and q each independently represent an integer of 1 or 2.

The component (A) is preferably one or more selected from the group consisting of sophorolipid and rhamnolipid, more preferably sophorolipid, from the viewpoint of securing excellent cleansing performance without being influenced by the hardness or temperature of water for use in cleansing.

In the case of using sophorolipid as the component (A), the content of the sophorolipid in the component (A) is preferably 50% by mass or more, more preferably 70% by mass or more, further more preferably 90% by mass or more, and may be 100% by mass.

In the case of using sophorolipid as the component (A), the mass ratio of the content of the lactonic form (LSL) to the content of the acidic form (ASL) (LSL/ASL) in the sophorolipid is preferably 5 or less, more preferably 3 or less, or the sophorolipid may not contain LSL, from the viewpoint of enhancing the fluidity of the cleansing composition.

In the case of using sophorolipid as the component (A), the mass ratio of the content of the lactonic form (LSL) to the content of the acidic form (ASL) (LSL/ASL) in the sophorolipid is preferably 2 or more, or the sophorolipid may not contain LSL, from the viewpoint of stably exerting excellent cleansing performance of the cleansing composition, and further, excellent suppression of protein elution.

The cleansing composition of the present invention comprises an internal olefin sulfonate having 8 or more and 24 or less carbon atoms, wherein a content of an internal olefin sulfonate having a sulfonic acid group at the 2-position is 40% by mass or less, as the component (B) together with the component (A). The component (B) can be obtained by sulfonating an internal olefin.

The number of carbon atoms in the component (B) is 8 or more, preferably 12 or more, more preferably 16 or more, and 24 or less, preferably 22 or less, more preferably 20 or less, further more preferably 18 or less, from the viewpoint of achieving both of cleansing performance and the stability of the cleansing composition. The number of carbon atoms in the component (B) is 8 or more and 24 or less, preferably 12 or more and 24 or less, more preferably 16 or more and 20 or less, further more preferably 16 or more and 18 or less.

In the internal olefin sulfonate as the component (B), the content of the internal olefin sulfonate having a sulfonic acid group at the 2-position is 40% by mass or less, preferably 35% by mass or less, more preferably 30% by mass or less, further more preferably 28% by mass or less, in the component (B) from the viewpoint of the stability of the cleansing composition. The content is preferably 10% by mass or more, further more preferably 15% by mass or more, from the viewpoint of the productivity of the component (B). The content of the internal olefin sulfonate having a sulfonic acid group at the 2-position is 40% by mass or less, preferably 10% by mass or more and 35% by mass or less, more preferably 10% by mass or more and 30% by mass or less, further more preferably 15% by mass or more and 28% by mass or less, in the component (B).

The component (B) preferably comprises an internal olefin sulfonate (IO-1S) having 8 or more and 24 or less carbon atoms and having a sulfonic acid group at the 2- or higher and 4- or lower position, and an internal olefin sulfonate (IO-2S) having 8 or more and 24 or less carbon atoms and having a sulfonic acid group at the 5- or higher position, from the viewpoint of achieving both of cleansing performance and the stability of the cleansing composition.

The mass ratio of the content of (IO-1S) to the content of (IO-2S) ((IO-1S)/(IO-2S)) in the component (B) is preferably 0.50 or more, more preferably 0.60 or more, further more preferably 0.70 or more, and preferably 6.5 or less, more preferably 6.0 or less, further more preferably 5.5 or less, even more preferably 5.0 or less, even more preferably 4.5 or less, even more preferably 4.0 or less, even more preferably 3.5 or less, even more preferably 3.0 or less, even more preferably 2.5 or less, even more preferably 2.0 or less, even more preferably 1.5 or less, from the above viewpoint. The mass ratio of the content of (IO-1S) to the content of (IO-2S) ((IO-1S)/(IO-2S)) in the component (B) is preferably 0.50 or more and 6.5 or less, more preferably 0.60 or more and 6.0 or less, further more preferably 0.70 or more and 5.5 or less, even more preferably 0.70 or more and 5.0 or less, even more preferably 0.70 or more and 4.5 or less, even more preferably 0.70 or more and 4.0 or less, even more preferably 0.70 or more and 3.5 or less, even more preferably 0.70 or more and 3.0 or less, even more preferably 0.70 or more and 2.5 or less, even more preferably 0.70 or more and 2.0 or less, even more preferably 0.70 or more and 1.5 or less.

The content of each compound having a distinct position of a sulfonic acid group in the component (B) can be measured with a high-performance liquid chromatography-mass spectrometer (hereinafter, referred to as HPLC-MS). The content of each compound having a distinct position of a sulfonic acid group according to the present invention is determined as a mass ratio based on the HPLC-MS peak area of the compound having a sulfonic acid group at each position in the total HAS of the component (B).

In this context, hydroxyalkane sulfonate, i.e., a hydroxy form of the internal olefin sulfonate, among compounds produced by the sulfonation of an internal olefin sulfonic acid is referred to as HAS. Thus, in the present invention, the internal olefin sulfonate (IO-1S) having 8 or more and 24 or less carbon atoms and having a sulfonic acid group at the 2- or higher and 4- or lower position means a sulfonate having 8 or more and 24 or less carbon atoms and having a sulfonic acid group at the 2- or higher and 4- or lower position in HAS forms having 8 or more and 24 or less carbon atoms.

The internal olefin sulfonate (IO-2S) having 8 or more and 24 or less carbon atoms and having a sulfonic acid

group at the 5- or higher position means a sulfonate having 8 or more and 24 or less carbon atoms and having a sulfonic acid group at the 5- or higher position in HAS forms having 8 or more and 24 or less carbon atoms.

In the internal olefin sulfonate (IO-2S) having 8 or more and 24 or less carbon atoms and having a sulfonic acid group at the 5- or higher position, the maximum value of the bonding position of the sulfonic acid group differs depending on the number of carbon atoms.

The mass ratio of the content of (IO-1S) to the content of (IO-2S) ((IO-1S)/(IO-2S)) is based on the finally obtained component (B). For example, even an internal olefin sulfonate obtained by mixing internal olefin sulfonates having a mass ratio ((IO-1S)/(IO-2S)) outside the above range is regarded as corresponding to the internal olefin sulfonate as the component (B) according to the present invention as long as the mass ratio ((IO-1S)/(IO-2S)) in the compositional profile of the internal olefin sulfonate falls within the above range.

The content of (IO-1S) in the component (B) is preferably 90% by mass or less, more preferably 85% by mass or less, further more preferably 80% by mass or less, and preferably 30% by mass or more, more preferably 40% by mass or more, from the viewpoint of achieving both of cleansing performance and the stability of the cleansing composition. The content of (IO-1S) in the component (B) is preferably 30% by mass or more and 90% by mass or less, more preferably 40% by mass or more and 85% by mass or less, further more preferably 40% by mass or more and 80% by mass or less.

Examples of the salt of the internal olefin sulfonate as the component (B) include alkali metal salts, alkaline earth metal (1/2 atom) salts, ammonium salts, and organic ammonium salts. Examples of the alkali metal salt include sodium salt and potassium salt. Examples of the organic ammonium salt include alkanol ammonium salts having 1 or more and 6 or less carbon atoms.

The component (B) according to the present invention is obtained, for example, by sulfonating an internal olefin having 8 or more and 24 or less carbon atoms, and can be obtained by using, as a starting material, an internal olefin in which the mass ratio of an olefin (IO-1) having 8 or more and 24 or less carbon atoms and having a double bond at the 1- or higher and 3- or lower position to an olefin (IO-2) having 8 or more and 24 or less carbon atoms and having a double bond at the 5- or higher position ((IO-1)/(IO-2)) in the internal olefin is 0.50 or more and 6.5 or less.

The internal olefin for obtaining the component (B) is constituted by an olefin (IO-1) having 8 or more and 24 or less carbon atoms and having a double bond at the 1- or higher and 3- or lower position, an olefin having 8 or more and 24 or less carbon atoms and having a double bond at the 4-position, and an olefin (IO-2) having 8 or more and 24 or less carbon atoms and having a double bond at the 5- or higher position. In the olefin (IO-2) having 8 or more and 24 or less carbon atoms and having a double bond at the 5- or higher position, the maximum value of the position of the double bond differs depending on the number of carbon atoms.

The mass ratio of the olefin (IO-1) having 8 or more and 24 or less carbon atoms and having a double bond at the 1- or higher and 3- or lower position to the olefin (IO-2) having 8 or more and 24 or less carbon atoms and having a double bond at the 5- or higher position ((IO-1)/(IO-2)) in the internal olefin having 8 or more and 24 or less carbon atoms is preferably 6.5 or less, more preferably 6.0 or less, further more preferably 5.5 or less, even more preferably 5.0 or less, even more preferably 4.5 or less, even more preferably 3.0 or less, even more preferably 2.5 or less, even more preferably 2.0 or less, even more preferably 1.5 or less, and preferably 0.50 or more, more preferably 0.55 or more, further more preferably 0.60 or more, from the viewpoint of cleansing performance. The mass ratio of the olefin (IO-1) having 8 or more and 24 or less carbon atoms and having a double bond at the 1- or higher and 3- or lower position to the olefin (IO-2) having 8 or more and 24 or less carbon atoms and having a double bond at the 5- or higher position ((IO-1)/(IO-2)) in the internal olefin having 8 or more and 24 or less carbon atoms is preferably 0.50 or more and 6.5 or less, more preferably 0.55 or more and 6.0 or less, further more preferably 0.60 or more and 5.5 or less, even more preferably 0.60 or more and 5.0 or less, even more preferably 0.60 or more and 4.5 or less, even more preferably 0.60 or more and 3.0 or less, even more preferably 0.60 or more and 2.5 or less, even more preferably 0.60 or more and 2.0 or less, even more preferably 0.60 or more and 1.5 or less.

The mass ratio ((IO-1)/(IO-2)) as to the internal olefin for obtaining the component (B) may be based on the finally obtained component (B). For example, even an internal olefin sulfonate obtained by using an olefin having a mass ratio ((IO-1)/(IO-2)) outside the above range as a starting material and further mixing the resulting internal olefin sulfonate can be regarded as corresponding to the internal olefin sulfonate as the component (B) obtained by using a predetermined olefin as a starting material as long as the mass ratio ((IO-1)/(IO-2)) in the compositional profile of the olefin corresponding to the starting material olefin falls within the above range.

The number of carbon atoms in the olefin serving as a starting material for the component (B) is preferably 8 or more, more preferably 12 or more, further more preferably 16 or more, from the viewpoint of achieving both of cleansing performance and the stability of the cleansing composition. The number of carbon atoms in the olefin serving as a starting material for the component (B) is preferably 24 or less, more preferably 22 or less, further more preferably 20 or less, further more preferably 18 or less, from the viewpoint of improving cleansing performance. The number of carbon atoms in the olefin serving as a starting material for the component (B) is preferably 8 or more and 24 or less, more preferably 12 or more and 22 or less, further more preferably 16 or more and 20 or less, further more preferably 16 or more and 18 or less.

The internal olefin serving as a starting material for the component (B) also includes an internal olefin containing a

trace amount of a so-called alpha olefin in which the position of a double bond is the 1-position of a carbon chain (hereinafter, also referred to as an $\alpha$-olefin). The content of the alpha olefin in the internal olefin is preferably 10% by mass or less, more preferably 5% by mass or less, further more preferably 3% by mass or less, and preferably 0.01% by mass or more, more preferably 0.05% by mass or more, from the viewpoint of the securing of stability of the composition in a low-temperature region, reduction in production cost, and improvement in productivity.

The internal olefin, when sulfonated, quantitatively produces B-sultone, and some of B-sultones are converted into $\gamma$-sultone and olefin sulfonic acid, which are further converted into hydroxyalkane sulfonate and olefin sulfonate in neutralization and hydrolysis steps (e.g., J. Am. Oil Chem. Soc. 69, 39 (1992)). In this context, the hydroxyl group of the hydroxyalkane sulfonate thus obtained resides inside the alkane chain, and the double bond of the olefin sulfonate resides inside the olefin chain. The resulting product is mainly their mixtures, some of which may contain a trace amount of hydroxyalkane sulfonate having a hydroxyl group at the end of the carbon chain or olefin sulfonate having a double bond at the end of the carbon chain.

In the present invention, each of these products and their mixture are collectively referred to as the internal olefin sulfonate (component (B)). The hydroxyalkane sulfonate is referred to as a hydroxy form of the internal olefin sulfonate (HAS), and the olefin sulfonate is referred to as an olefin form of the internal olefin sulfonate (hereinafter, also referred to as IOS).

The mass ratio of a compound in the component (B) can be measured with HPLC-MS. Specifically, the mass ratio can be determined from the HPLC-MS peak area of the component (B).

The distribution of double bonds in the internal olefin can be measured with, for example, a gas chromatograph-mass spectrometer (hereinafter, abbreviated to GC-MS). Specifically, each of components differing in carbon chain length and double bond position is accurately separated with a gas chromatograph analyzer (hereinafter, abbreviated to GC), and each component can be applied to a mass spectrometer (hereinafter, abbreviated to MS) to identify the position of the double bond. The proportion of each component can be determined from the resulting GC peak area. A value determined from the GC peak area is used as the content of each olefin having a double bond at the specific position. In the case of using a mixture of olefins differing in the number of carbon atoms, the distribution of double bond positions is indicated as the distribution of double bond positions in olefins having the same number of carbon atoms.

In the present invention, in the case of using a mixture of a plurality of internal olefin sulfonates obtained from a plurality of starting material olefins differing in double bond position, the distribution of double bond positions in the olefins serving as starting materials for the internal olefin sulfonates is calculated as a distribution in olefins having the same number of carbon atoms. Specifically, for example, an average double bond position of starting material olefins having 16 carbon atoms is a value determined according to the following expression (6), and an average double bond position of starting material olefins having 18 carbon atoms is a value determined according to the following expression (7).

$$\textbf{Average double bond position of starting material olefins having 16 carbon atoms} = \sum_{n=1}^{8} n \times C_n/100 \quad \ldots \textbf{(6)}$$

$$\textbf{Average double bond position of starting material olefins having 18 carbon atoms} = \sum_{n=1}^{9} n \times C_n/100 \quad \ldots \textbf{(7)}$$

The mass ratio of the content of the component (A) to the content of the component (B) ((A)/(B)) is preferably 0.0050 or more, more preferably 0.050 or more, further more preferably 0.10 or more, even more preferably 0.20 or more, from the viewpoint of stably exerting excellent cleansing performance without being influenced by the hardness or temperature of water for use in cleansing. The mass ratio of the content of the component (A) to the content of the component (B) ((A)/(B)) is preferably 95.00 or less, more preferably 19.00 or less, further more preferably 9.00 or less, further more preferably 4.00 or less, more preferably 2.00 or less, even more preferably 1.00 or less, from the viewpoint of excellent colorless transparency. The mass ratio of the content of the component (A) to the content of the component (B) ((A)/(B)) is preferably 0.0050 or more and 95.00 or less, more preferably 0.050 or more and 19.00 or less, further more preferably 0.10 or more and 9.00 or less, further more preferably 0.20 or more and 4.00 or less, further more preferably 0.20 or more and 2.00 or less, even more preferably 0.20 or more and 1.00 or less.

The mass ratio of the content of the component (A) to the content of the component (B) ((A)/(B)) is preferably 0.0050 or more, more preferably 0.050 or more, further more preferably 0.10 or more, even more preferably 0.20 or more, even more preferably 0.30 or more, even more preferably 0.40 or more, and preferably 95.00 or less, more preferably 19.00 or less, further more preferably 9.00 or less, further more preferably 4.00 or less, more preferably 2.00 or less, even more preferably 1.00 or less, from the viewpoint of the suppression of protein elution.

More specifically, when a hard-surface article is to be cleansed with the cleansing composition of the present

invention, the mass ratio of the content of the component (A) to the content of the component (B) ((A)/(B)) is preferably 0.33 or more, more preferably 0.67 or more, and preferably 1.00 or less, from the viewpoint of excellent cleansing performance, colorless transparency, fluidity, and suppression of protein elution. When a hard-surface article is to be cleansed with the cleansing composition of the present invention, the mass ratio of the content of the component (A) to the content of the component (B) ((A)/(B)) is preferably 0.33 or more and 1.00 or less, more preferably 0.67 or more and 1.00 or less.

When the body is to be cleansed with the cleansing composition of the present invention, the mass ratio of the content of the component (A) to the content of the component (B) ((A)/(B)) is preferably 0.33 or more, more preferably 0.67 or more, and preferably 1.00 or less, from the viewpoint of excellent colorless transparency, fluidity, and suppression of protein elution. When the body is to be cleansed with the cleansing composition of the present invention, the mass ratio of the content of the component (A) to the content of the component (B) ((A)/(B)) is preferably 0.33 or more and 1.00 or less, more preferably 0.67 or more and 1.00 or less.

When an apparel product is to be cleansed with the cleansing composition of the present invention, the mass ratio of the content of the component (A) to the content of the component (B) ((A)/(B)) is preferably 0.33 or more and 0.67 or less, from the viewpoint of excellent cleansing performance, fluidity, and colorless transparency.

The mass ratio of the content of the component (A) to the total content of the component (A) and the component (B) ((A)/{(A) + (B)}) is preferably 0.0050 or more, more preferably 0.010 or more, further more preferably 0.10 or more, even more preferably 0.20 or more, from the viewpoint of stably exerting excellent cleansing performance without being influenced by the hardness or temperature of water for use in cleansing. The mass ratio of the content of the component (A) to the total content of the component (A) and the component (B) ((A)/{(A) + (B)}) is preferably 0.99 or less, more preferably 0.95 or less, further more preferably 0.90 or less, further more preferably 0.80 or less, further more preferably 0.70 or less, further more preferably 0.60 or less, even more preferably 0.50 or less, from the viewpoint of excellent colorless transparency. The mass ratio of the content of the component (A) to the total content of the component (A) and the component (B) ((A)/{(A) + (B)}) is preferably 0.0050 or more and 0.99 or less, more preferably 0.010 or more and 0.95 or less, further more preferably 0.10 or more and 0.90 or less, further more preferably 0.20 or more and 0.80 or less, further more preferably 0.20 or more and 0.70 or less, further more preferably 0.20 or more and 0.60 or less, even more preferably 0.20 or more and 0.50 or less.

The mass ratio of the content of the component (A) to the total content of the component (A) and the component (B) ((A)/{(A) + (B)}) is preferably 0.0050 or more, more preferably 0.010 or more, further more preferably 0.10 or more, even more preferably 0.20 or more, even more preferably 0.30 or more, and preferably 0.99 or less, more preferably 0.95 or less, further more preferably 0.90 or less, further more preferably 0.80 or less, further more preferably 0.70 or less, further more preferably 0.60 or less, even more preferably 0.50 or less, from the viewpoint of the suppression of protein elution.

More specifically, when a hard-surface article is to be cleansed with the cleansing composition of the present invention, the mass ratio of the content of the component (A) to the total content of the component (A) and the component (B) ((A)/{(A) + (B)}) is preferably 0.25 or more, more preferably 0.40 or more, and preferably 0.50 or less, from the viewpoint of excellent cleansing performance, colorless transparency, and suppression of protein elution. When a hard-surface article is to be cleansed with the cleansing composition of the present invention, the mass ratio of the content of the component (A) to the total content of the component (A) and the component (B) ((A)/{(A) + (B)}) is preferably 0.25 or more and 0.50 or less, more preferably 0.40 or more and 0.50 or less.

When the body is to be cleansed with the cleansing composition of the present invention, the mass ratio of the content of the component (A) to the total content of the component (A) and the component (B) ((A)/{(A) + (B)}) is preferably 0.25 or more, more preferably 0.40 or less, and preferably 0.50 or less, from the viewpoint of excellent colorless transparency, fluidity, and suppression of protein elution. When the body is to be cleansed with the cleansing composition of the present invention, the mass ratio of the content of the component (A) to the total content of the component (A) and the component (B) ((A)/{(A) + (B)}) is preferably 0.25 or more and 0.50 or less, more preferably 0.40 or more and 0.50 or less.

When an apparel product is to be cleansed with the cleansing composition of the present invention, the mass ratio of the content of the component (A) to the total content of the component (A) and the component (B) ((A)/{(A) + (B)}) is preferably 0.25 or more and 0.40 or less from the viewpoint of excellent cleansing performance, fluidity, and colorless transparency.

The total content of the component (A) and the component (B) in the cleansing composition of the present invention can be 0.15% by mass or more and is preferably 1.5% by mass or more, more preferably 2% by mass or more, further more preferably 3% by mass or more, from the viewpoint of stably exerting excellent cleansing performance without being influenced by the hardness or temperature of water for use in cleansing, and from the viewpoint of sufficiently exerting surface activity by the component (A) and the component (B), and eliminating the need of using other components which interfere with cleansing performance. The total content of the component (A) and the component (B) in the cleansing composition of the present invention may be 55% by mass or less, may also be 50% by mass or less, and may further be 45% by mass or less, or the total content of the component (A) and the component (B) is preferably 100% by mass or less in the cleansing composition of the present invention, from the viewpoint of cost optimization and from the viewpoint of excellent rinsability.

More specifically, when a hard-surface article or a body is to be cleansed with the cleansing composition of the present invention, or in the case of a so-called low-concentration-type cleansing composition, for example, the case of filling a container such as a spray container or a pump foamer with the cleansing composition, and spraying or discharging the cleansing composition from the container for use in cleansing, the total content of the component (A) and the component (B) is preferably 0.15% by mass or more, more preferably 1.5% by mass or more, further more preferably 2% by mass or more, even more preferably 3% by mass or more, in the cleansing composition of the present invention from the viewpoint of stably exerting excellent cleansing performance without being influenced by the hardness or temperature of water for use in cleansing, and from the viewpoint of sufficiently exerting surface activity by the component (A) and the component (B), and eliminating the need of using other components which interfere with cleansing performance. When the cleansing composition of the present invention is a low-concentration-type cleansing composition, the total content of the component (A) and the component (B) is preferably 25% by mass or less, more preferably 20% by mass or less, further more preferably 15% by mass or less, even more preferably 10% by mass or less, in the cleansing composition of the present invention from the viewpoint of cost optimization and from the viewpoint of excellent rinsability. When the cleansing composition of the present invention is a low-concentration-type cleansing composition, the total content of the component (A) and the component (B) is preferably 0.15% by mass or more and 25% by mass or less, more preferably 1.5% by mass or more and 20% by mass or less, further more preferably 2% by mass or more and 15% by mass or less, even more preferably 3% by mass or more and 10% by mass or less, in the cleansing composition of the present invention.

When an apparel product is to be cleansed with the cleansing composition of the present invention, or in the case of a so-called high-concentration-type cleansing composition, the total content of the component (A) and the component (B) is preferably 14% by mass or more, more preferably 16% by mass or more, further more preferably 18% by mass or more, even more preferably 20% by mass or more, in the cleansing composition of the present invention from the viewpoint of stably exerting excellent cleansing performance without being influenced by the hardness or temperature of water for use in cleansing, and from the viewpoint of sufficiently exerting surface activity by the component (A) and the component (B), and eliminating the need of using other components which interfere with cleansing performance. When the cleansing composition of the present invention is a high-concentration-type cleansing composition, the total content of the component (A) and the component (B) is preferably 55% by mass or less, more preferably 50% by mass or less, further more preferably 45% by mass or less, even more preferably 40% by mass or less, in the cleansing composition of the present invention from the viewpoint of cost optimization, from the viewpoint of excellent rinsability, and further, from the viewpoint of ensuring the fluidity of the cleansing composition. When the cleansing composition of the present invention is a high-concentration-type cleansing composition, the total content of the component (A) and the component (B) is preferably 14% by mass or more and 55% by mass or less, more preferably 16% by mass or more and 50% by mass or less, further more preferably 18% by mass or more and 45% by mass or less, even more preferably 20% by mass or more and 40% by mass or less, in the cleansing composition of the present invention.

The content of the component (A) can be 0.01% by mass or more and is preferably 0.04% by mass or more, more preferably 0.4% by mass or more, further more preferably 1% by mass or more, in the cleansing composition of the present invention from the viewpoint of stably exerting excellent cleansing performance without being influenced by the hardness or temperature of water for use in cleansing. The content of the component (A) may be 50% by mass or less, may also be 40% by mass or less, may further be 30% by mass or less, may further be 20% by mass or less, may further be 15% by mass or less, and may still further be 10% by mass or less, in the cleansing composition of the present invention from the viewpoint of excellent colorless transparency, from the viewpoint of cost optimization, and further, from the viewpoint of excellent rinsability.

More specifically, when a hard-surface article or a body is to be cleansed with the cleansing composition of the present invention, or in the case of a so-called low-concentration-type cleansing composition, for example, the case of filling a container such as a spray container or a pump foamer with the cleansing composition, and spraying or discharging the cleansing composition from the container for use in cleansing, the content of the component (A) is preferably 0.01% by mass or more, more preferably 0.04% by mass or more, further more preferably 0.4% by mass or more, even more preferably 1% by mass or more, in the cleansing composition of the present invention from the viewpoint of stably exerting excellent cleansing performance without being influenced by the hardness or temperature of water for use in cleansing. When the cleansing composition of the present invention is a low-concentration-type cleansing composition, the content of the component (A) is preferably 10% by mass or less, more preferably 5% by mass or less, further more preferably 3% by mass or less, even more preferably 2.3% by mass or less, even more preferably 1.5% by mass or less, in the cleansing composition of the present invention from the viewpoint of excellent colorless transparency, from the viewpoint of cost optimization, and from the viewpoint of excellent rinsability. When the cleansing composition of the present invention is a low-concentration-type cleansing composition, the content of the component (A) is preferably 0.01% by mass or more and 10% by mass or less, more preferably 0.04% by mass or more and 5% by mass or less, further more preferably 0.4% by mass or more and 3% by mass or less, further more preferably 1% by mass or more and 2.3% by mass or less, even more preferably 1% by mass or more and 1.5% by mass or less, in the cleansing composition of the present invention.

When an apparel product is to be cleansed with the cleansing composition of the present invention, or in the case

of a so-called high-concentration-type cleansing composition, the content of the component (A) is preferably 1% by mass or more, more preferably 2% by mass or more, further more preferably 4% by mass or more, even more preferably 5% by mass or more, in the cleansing composition of the present invention from the viewpoint of stably exerting excellent cleansing performance without being influenced by the hardness or temperature of water for use in cleansing. When the cleansing composition of the present invention is a high-concentration-type cleansing composition, the content of the component (A) is preferably 50% by mass or less, more preferably 40% by mass or less, further more preferably 30% by mass or less, further more preferably 20% by mass or less, further more preferably 15% by mass or less, even more preferably 10% by mass or less, in the cleansing composition of the present invention from the viewpoint of excellent colorless transparency, from the viewpoint of cost optimization, and further, from the viewpoint of excellent rinsability. When the cleansing composition of the present invention is a high-concentration-type cleansing composition, the content of the component (A) is preferably 1% by mass or more and 50% by mass or less, more preferably 2% by mass or more and 40% by mass or less, further more preferably 4% by mass or more and 30% by mass or less, further more preferably 5% by mass or more and 20% by mass or less, further more preferably 5% by mass or more and 15% by mass or less, even more preferably 5% by mass or more and 10% by mass or less, in the cleansing composition of the present invention.

The content of the component (B) can be 0.1% by mass or more and is preferably 1% by mass or more, more preferably 1.5% by mass or more, further more preferably 2.6% by mass or more, even more preferably 3.5% by mass or more, in the cleansing composition of the present invention from the viewpoint of stably exerting excellent cleansing performance without being influenced by the hardness or temperature of water for use in cleansing. The content of the component (B) may be 50% by mass or less, may also be 40% by mass or less, may further be 35% by mass or less, may still further be 30% by mass or less, in the cleansing composition of the present invention from the viewpoint of cost optimization and from the viewpoint of excellent rinsability.

More specifically, when a hard-surface article or a body is to be cleansed with the cleansing composition of the present invention, or in the case of a so-called low-concentration-type cleansing composition, for example, the case of filling a container such as a spray container or a pump foamer with the cleansing composition, and spraying or discharging the cleansing composition from the container for use in cleansing, the content of the component (B) is preferably 0.1% by mass or more, more preferably 1% by mass or more, further more preferably 1.5% by mass or more, further more preferably 2.6% by mass or more, even more preferably 3.5% by mass or more, in the cleansing composition of the present invention from the viewpoint of stably exerting excellent cleansing performance without being influenced by the hardness or temperature of water for use in cleansing. When the cleansing composition of the present invention is a low-concentration-type cleansing composition, the content of the component (B) is preferably 20% by mass or less, more preferably 10% by mass or less, further more preferably 5% by mass or less, even more preferably 4% by mass or less, from the viewpoint of cost optimization and from the viewpoint of excellent rinsability. When the cleansing composition of the present invention is a low-concentration-type cleansing composition, the content of the component (B) is preferably 0.1% by mass or more and 20% by mass or less, more preferably 1% by mass or more and 10% by mass or less, further more preferably 1.5% by mass or more and 5% by mass or less, further more preferably 2.6% by mass or more and 4% by mass or less, even more preferably 3.5% by mass or more and 4% by mass or less, in the cleansing composition of the present invention.

When an apparel product is to be cleansed with the cleansing composition of the present invention, or in the case of appropriately diluting the cleansing composition with water for use in cleansing, i.e., in the case of a so-called high-concentration-type cleansing composition, the content of the component (B) is preferably 1% by mass or more, more preferably 5% by mass or more, further more preferably 8% by mass or more, further more preferably 10% by mass or more, further more preferably 20% by mass or more, even more preferably 25% by mass or more, in the cleansing composition of the present invention from the viewpoint of stably exerting excellent cleansing performance without being influenced by the hardness or temperature of water for use in cleansing. When the cleansing composition of the present invention is a high-concentration-type cleansing composition, the content of the component (B) is preferably 50% by mass or less, more preferably 40% by mass or less, further more preferably 35% by mass or less, even more preferably 30% by mass or less, in the cleansing composition of the present invention from the viewpoint of cost optimization and from the viewpoint of excellent rinsability. When the cleansing composition of the present invention is a high-concentration-type cleansing composition, the content of the component (B) is preferably 1% by mass or more and 50% by mass or less, more preferably 5% by mass or more and 40% by mass or less, further more preferably 8% by mass or more and 35% by mass or less, further more preferably 10% by mass or more and 30% by mass or less, further more preferably 20% by mass or more and 30% by mass or less, even more preferably 25% by mass or more and 30% by mass or less, in the cleansing composition of the present invention.

The internal olefin sulfonate as the component (B) is obtained by sulfonating the starting material olefin through reaction with sulfur trioxide and specifically, can be obtained by sulfonating the starting material olefin, followed by neutralization and subsequent hydrolysis.

More specifically, the amount of the sulfur trioxide used in sulfonating the starting material olefin is preferably 0.8 mol or more, more preferably 0.9 mol or more, further more preferably 0.95 mol or more, per mol of the starting material

olefin from the viewpoint of improving the yield of the component (B) and from the viewpoint of improving reactivity. The amount of the sulfur trioxide used in sulfonating the starting material olefin is preferably 1.2 mol or less, more preferably 1.1 mol or less, further more preferably 1.05 mol or less, from the viewpoint of economic performance and from the viewpoint of securing colorless transparency. The amount of the sulfur trioxide used in sulfonating the starting material olefin is preferably 0.8 mol or more and 1.2 mol or less, more preferably 0.9 mol or more and 1.1 mol or less, even more preferably 0.95 mol or more and 1.05 mol or less, per mol of the starting material olefin.

The reaction temperature in sulfonating the starting material olefin is preferably 0°C or higher from the viewpoint of preventing the coagulation of the sulfur trioxide and component (B), and preferably 50°C or lower from the viewpoint of securing colorless transparency. The reaction temperature in sulfonating the starting material olefin is preferably 0°C or higher and 50°C or lower.

For neutralization, an alkali compound such as sodium hydroxide, potassium hydroxide, ammonia, or 2-aminoethanol is reacted. The amount of the alkali compound added is preferably a 1.0-fold or more molar amount, more preferably a 1.03-fold or more molar amount, per mol of a sulfonic acid group from the viewpoint of preventing the formation of impurities such as the starting material olefin or inorganic salts, and from the viewpoint of improving reactivity. The amount of the alkali compound added is preferably a 2.5-fold or less molar amount, more preferably a 2.0-fold or less molar amount, further more preferably a 1.5-fold or less molar amount, per mol of a sulfonic acid group from the viewpoint of economic performance and from the viewpoint of preventing the formation of impurities such as the starting material olefin or inorganic salts. The amount of the alkali compound added is preferably a 1.0-fold or more molar amount and 2.5-fold or less molar amount, more preferably a 1.03 mol-fold or more and 2.0-fold or less molar amount, even more preferably a 1.03 mol-fold or more and 1.5-fold or less molar amount, per mol of a sulfonic acid group.

For neutralization, the temperature in mixing the sulfonated starting material olefin with the alkali compound, and the reaction temperature are preferably 40°C or lower, more preferably 35°C or lower, further more preferably 30°C or lower, even more preferably 25°C or lower, from the viewpoint of preventing the formation of impurities such as the internal olefin or inorganic salts ascribable to side reaction, and preferably 0°C or higher, more preferably 10°C or higher, further more preferably 15°C or higher, even more preferably 20°C or higher, from the viewpoint of improving reactivity. The temperature in mixing the sulfonated starting material olefin with the alkali compound, and the reaction temperature are preferably 0°C or higher and 40°C or lower, more preferably 10°C or higher and 35°C or lower, further more preferably 15°C or higher and 30°C or lower, even more preferably 20°C or higher and 25°C or lower.

The reaction temperature in hydrolysis which is performed after neutralization is preferably 120°C or higher, more preferably 140°C or higher, further more preferably 160°C or higher, in the presence of water from the viewpoint of improving reactivity. The reaction temperature in hydrolysis is preferably 220°C or lower, more preferably 180°C or lower, from the viewpoint of suppressing the degradation of a product. The reaction temperature in hydrolysis is preferably 120°C or higher and 220°C or lower, more preferably 140°C or higher and 180°C or lower, further more preferably 160°C or higher and 180°C or lower.

The reaction time in hydrolysis is preferably 30 minutes or longer, more preferably 45 minutes or longer, from the viewpoint of completing the reaction. The reaction time in hydrolysis is preferably 240 minutes or shorter, more preferably 180 minutes or shorter, further more preferably 120 minutes or shorter, even more preferably 90 minutes or shorter, from the viewpoint of improving productivity. The reaction time in hydrolysis is preferably 30 minutes or longer and 240 minutes or shorter, more preferably 45 minutes or longer and 180 minutes or shorter, even more preferably 45 minutes or longer and 120 minutes or shorter, even more preferably 45 minutes or longer and 90 minutes or shorter. These reactions can be continuously performed. After the completion of reaction, the resulting product can be purified by extraction, washing, or the like.

The cleansing composition of the present invention comprises water (C). The water according to the present invention means total water contained in the cleansing composition, including not only purified water or the like blended into the cleansing composition, but also water contained in each component blended thereinto. The water thus contained favorably dissolves or disperses each of the contained components so that desired effects can be sufficiently exerted.

The content of the component (C) may be 30% by mass or more and may be 99% by mass or less, in the cleansing composition of the present invention.

More specifically, when a hard-surface article or a body is to be cleansed with the cleansing composition of the present invention, or in the case of a so-called low-concentration-type cleansing composition, for example, the case of filling a container such as a spray container or a pump foamer with the cleansing composition, and spraying or discharging the cleansing composition from the container for use in cleansing, the content of the component (C) is preferably 70% by mass or more, more preferably 75% by mass or more, further more preferably 80% by mass or more, even more preferably 85% by mass or more, in the cleansing composition of the present invention from the viewpoint of cost optimization and from the viewpoint of excellent rinsability. When the cleansing composition of the present invention is a low-concentration-type cleansing composition, the content of the component (C) is preferably 99% by mass or less, more preferably 98% by mass or less, further more preferably 97% by mass or less, even more preferably 95% by mass or less, in the cleansing composition of the present invention from the viewpoint of stably exerting excellent cleansing performance. When the

cleansing composition of the present invention is a low-concentration-type cleansing composition, the content of the component (C) is preferably 70% by mass or more and 99% by mass or less, more preferably 75% by mass or more and 98% by mass or less, further more preferably 80% by mass or more and 97% by mass or less, even more preferably 85% by mass or more and 95% by mass or less, in the cleansing composition of the present invention.

When an apparel product is to be cleansed with the cleansing composition of the present invention, or in the case of appropriately diluting the cleansing composition with water for use in cleansing, i.e., in the case of a so-called high-concentration-type cleansing composition, the content of the component (C) is preferably 20% by mass or more, more preferably 30% by mass or more, further more preferably 35% by mass or more, even more preferably 40% by mass or more, and preferably 85% by mass or less, more preferably 80% by mass or less, further more preferably 75% by mass or less, even more preferably 70% by mass or less, in the cleansing composition of the present invention from the viewpoint of cost optimization, from the viewpoint of excellent rinsability, and from the viewpoint of the fluidity of the cleansing composition. When the cleansing composition of the present invention is a high-concentration-type cleansing composition, the content of the component (C) is preferably 20% by mass or more and 85% by mass or less, more preferably 30% by mass or more and 80% by mass or less, further more preferably 35% by mass or more and 75% by mass or less, even more preferably 40% by mass or more and 70% by mass or less, in the cleansing composition of the present invention.

The cleansing composition of the present invention may comprise an additional surfactant other than the component (A) and the component (B) without inhibiting the advantageous effects of the present invention.

Examples of the surfactant include anionic surfactants, cationic surfactants, amphoteric surfactants, and nonionic surfactants, other than the component (A) and the component (B).

Specific examples of the anionic surfactant include one or more selected from the group consisting of:

a sulfonate, other than the component (B), selected from the group consisting of aminoethyl sulfonate such as N-acyl methyl taurine salt, alkylbenzene sulfonate, alkenylbenzene sulfonate, alkane sulfonate, and $\alpha$-olefin sulfonate having 14 carbon atoms;
an amino acid salt selected from the group consisting of acyl glutamate, sarcosine derivatives, alanine derivatives, glycine derivatives, and arginine derivatives;
a sulfosuccinate selected from the group consisting of sulfosuccinic acid alkyl ester salts, and polyoxyalkylene sulfosuccinic acid alkyl ester salts;
a sulfuric acid ester salt selected from the group consisting of alkyl sulfate, alkenyl sulfate, polyoxyalkylene alkyl ether sulfate, polyoxyalkylene alkenyl ether sulfate, and polyoxyalkylene alkyl phenyl ether sulfate; and
a carboxylate selected from the group consisting of fatty acid salts and polyoxyalkylene alkyl ether acetate.

Examples of the cationic surfactant include one or more selected from the group consisting of: quaternary ammonium salts having a hydrocarbon group having 12 or more and 28 or less carbon atoms, which may be interrupted with an amide group, an ester group or an ether group; pyridinium salts; and salts of mineral acids or organic acids of tertiary amine.

Specific examples thereof include one or more selected from the group consisting of: mono-long-chain alkyl trimethylammonium salts such as octyl trimethylammonium salt, decyl trimethylammonium salt, lauryl trimethylammonium salt, myristyl trimethylammonium salt, cetyl trimethylammonium salt, stearyl trimethylammonium salt, behenyl trimethylammonium salt, and octadecyloxypropyl trimethylammonium salt; di-long-chain alkyl dimethylammonium salts such as dioctyl trimethylammonium salt, didecyl trimethylammonium salt, dilauryl trimethylammonium salt, dimyristyl trimethylammonium salt, dicetyl trimethylammonium salt, distearyl dimethylammonium salt, and diisotetradecyl dimethylammonium salt; and mono-long-chain alkyl dimethylamine salts such as hydrochloride, citrate, or lactate of stearyl dimethylamine, behenyl dimethylamine, octadecyloxypropyl dimethylamine, and dimethylaminopropylstearamide.

Specific examples of the amphoteric surfactant include one or more selected from the group consisting of carbobetaine and sulfobetaine having an alkyl group, an alkenyl group, or an acyl group having 6 or more and 22 or less carbon atoms, preferably 8 or more and 18 or less carbon atoms.

Specific examples of the nonionic surfactant include one or more selected from the group consisting of: polyoxyalkylene alkyl ether having 6 or more and 22 or less carbon atoms in the alkyl group; fatty acid alkanol amide such as mono- or di-alkanolamide having 6 or more and 22 or less carbon atoms in the fatty acid; chemically synthesized alkyl glycoside having 6 or more and 22 or less carbon atoms in the alkyl group; and alkyl glyceryl ether having 6 or more and 22 or less carbon atoms in the alkyl group.

The content of the additional surfactant other than the component (A) and the component (B) in the cleansing composition of the present invention is preferably 10% by mass or less, more preferably 5% by mass or less, or the cleansing composition of the present invention may not contain the additional surfactant other than the component (A) and the component (B), from the viewpoint of preventing the formation of unnecessary deposits and reduction in cleansing performance.

More specifically, when a hard-surface article or a body is to be cleansed with the cleansing composition of the

present invention, or in the case of a so-called low-concentration-type cleansing composition, for example, the case of filling a container such as a spray container or a pump foamer with the cleansing composition, and spraying or discharging the cleansing composition from the container for use in cleansing, the total content of the component (A) and the component (B) per 100% by mass as the total content of all the surfactants of the component (A), the component (B), and the additional surfactant other than the component (A) and the component (B) is preferably 10% by mass or more, more preferably 20% by mass or more, further more preferably 30% by mass or more, even more preferably 50% by mass or more, and preferably 100% by mass or less, in the cleansing composition of the present invention from the viewpoint of preventing the formation of unnecessary deposits and reduction in cleansing performance.

When an apparel product is to be cleansed with the cleansing composition of the present invention, or in the case of appropriately diluting the cleansing composition with water for use in cleansing, i.e., in the case of a so-called high-concentration-type cleansing composition, the total content of the component (A) and the component (B) per 100% by mass as the total content of all the surfactants of the component (A), the component (B), and the additional surfactant other than the component (A) and the component (B) is preferably 10% by mass or more, more preferably 20% by mass or more, further more preferably 30% by mass or more, even more preferably 50% by mass or more, preferably 100% by mass or less, in the cleansing composition of the present invention from the viewpoint of preventing the formation of unnecessary deposits and reduction in cleansing performance.

The cleansing composition of the present invention can have a limited content of a chelating agent. Usually, the chelating agent is used, for example, for suppressing reduction in cleansing performance caused by variations in hardness of water for use in cleansing. Nonetheless, the blending of the chelating agent could be responsible for forming unnecessary deposits in cleansing compositions. The cleansing composition of the present invention, however, is capable of having a limited content of such a chelating agent because of retaining excellent cleansing performance without being influenced by the hardness or temperature of water.

Specific examples of the chelating agent include one or more selected from the group consisting of: condensed phosphates such as tripolyphosphate, pyrophosphate, and orthophosphate; aluminosilicates or synthetic lamellar crystalline silicates, such as zeolite; organic acid salts such as citrate, isocitrate, nitrilotriacetate, and ethylenediaminetetraacetate; and polyacetal carboxylates.

The content of the chelating agent is preferably 10% by mass or less, more preferably 5% by mass or less, further more preferably 1% by mass or less, in the cleansing composition of the present invention, or the cleansing composition of the present invention may not contain the chelating agent.

The cleansing composition of the present invention can limit content of a solvent other than the water, which might result in a complicated production process or increased environmental load, though used for securing favorable stability of the composition.

Specific examples of the solvent other than the water include one or more selected from the group consisting of:

amide compounds such as N,N-dimethylformamide and 3-methoxy-N,N-dimethylpropionamide;
alkyl ethers and aryl ethers of polyhydric alcohols, such as ethylene glycol monophenyl ether, ethylene glycol monoallyl ether, diethylene glycol monophenyl ether, diethylene glycol monobutyl ether, propylene glycol monobutyl ether, and tetraethylene glycol chlorophenyl ether;
polyhydric alcohols such as 3-methyl-1,3-propanediol, 3-methyl-1,3-butanediol, 3,3-dimethyl-1,2-butanediol, 2,2-diethyl-1,3-propanediol, 2-methyl-2-propyl-1,3-propanediol, 2,4-dimethyl-2,4-pentanediol, 2,5-dimethyl-2,5-hexanediol, 5-hexene-1,2-diol, 2-ethyl-1,3-hexanediol, 2, 2, 4-trimethyl-1, 3-pentanediol, ethylene glycol, propylene glycol, butylene glycol, and glycerin;
monohydric alcohols such as ethanol, isopropyl alcohol, butanol, pentanol, and hexanol; and
aromatic alcohols such as benzyl alcohol.

The content of the solvent other than the water is preferably 30% by mass or less, more preferably 20% by mass or less, further more preferably 10% by mass or less, in the cleansing composition of the present invention, or the cleansing composition of the present invention may not contain the solvent other than the water.

The cleansing composition of the present invention can contain, in addition to the components described above, a viscosity reducing agent, an antiseptic, and a reducing agent as well as other components which are used as usual starting materials for cleansing agents, without inhibiting the advantageous effects of the present invention. Examples of the component include feel improving agents, thickeners, fragrances, ultraviolet absorbers, visible light absorbers, antioxidants, colorants, antiseptics, pH adjusters, viscosity adjusters, pearlizing agents, and humectants.

The pH at 25°C of the cleansing composition of the present invention is preferably 3 or higher, more preferably 4 or higher, from the viewpoint of effectively suppressing unnecessary hydrolysis or the like of the component (A), and securing excellent cleansing performance, and preferably 13.5 or lower from the viewpoint of securing excellent cleansing performance, and ensuring the antiseptic properties of the composition. The pH at 25°C of the cleansing composition of the present invention is preferably 3 or higher and 13.5 or lower, more preferably 4 or higher and 13.5 or lower.

The pH at 25°C of the cleansing composition of the present invention means a value measured using a pH electrode.

The cleansing composition of the present invention can be diluted with water to prepare a cleansing solution for cleansing a hard-surface article, an apparel product, or a body (hereinafter, also referred to as the cleansing solution of the present invention). The hardness of the water for use in dilution can be 0.1 °DH or more, may be 1 °DH or more, may also be 2 °DH or more, and may further be 3 °DH or more, and is usually 30 °DH or less, may be 25 °DH or less, and may further be 20 °DH or less, because the cleansing composition of the present invention exerts excellent cleansing performance without being influenced of the hardness of the water.

The unit "°DH" is a German degree Hardness and is regarded as 1 °DH when 10 g (= 10 mg/L) of calcium oxide is contained per cubic meter of water. In the present invention, the unit "°DH" means a value calculated from a mass concentration when the total molar concentration of a calcium ion and a magnesium ion present in the water for use in dilution is converted to the same molar concentration of calcium oxide.

The total content of the component (A) and the component (B) in the cleansing solution of the present invention is preferably $5 \times 10^{-5}$% by mass or more, more preferably $1 \times 10^{-3}$% by mass or more, further more preferably $5 \times 10^{-3}$% by mass or more, even more preferably $1.5 \times 10^{-2}$% by mass or more, in the cleansing solution of the present invention from the viewpoint of retaining excellent cleansing performance. The upper limit value may be 10% by mass or less, though it is not particularly limited.

In order to prepare the cleansing solution by diluting the cleansing composition of the present invention with water, the dilution ratio of the cleansing composition may be 1.5-fold or more, may be 2-fold or more, or may be 3-fold or more, from the viewpoint of excellent rinsability after cleansing, and may be 10 000-fold or less, may be 5 000-fold or less, or may be 2 000-fold or less, from the viewpoint of exerting excellent cleansing performance. In order to prepare the cleansing solution by diluting the cleansing composition of the present invention with water, the dilution ratio of the cleansing composition may be 1.5-fold or more and 10 000-fold or less, may be 2-fold or more and 5 000-fold or less, or may be 3-fold or more and 2 000-fold or less.

More specifically, when a hard-surface article or a body is to be cleansed with the cleansing solution of the present invention, or in the case of a cleansing solution prepared by diluting, with water, a so-called low-concentration-type cleansing composition, for example, the case of filling a container such as a spray container or a pump foamer with the cleansing solution, and spraying or discharging the cleansing solution from the container for use in cleansing, the dilution ratio of the cleansing composition is preferably 1-fold or more, more preferably 2-fold or more, further more preferably 3-fold or more, from the viewpoint of excellent rinsability after cleansing and from the viewpoint of a low residual amount on the surface to be cleansed, and preferably 100-fold or less, more preferably 50-fold or less, further more preferably 10-fold or less, from the viewpoint of exerting excellent foamability and cleansing performance. When a hard-surface article or a body is to be cleansed with the cleansing solution of the present invention, or in the case of a cleansing solution prepared by diluting, with water, a so-called low-concentration-type cleansing composition, for example, the case of filling a container such as a spray container or a pump foamer with the cleansing solution, and spraying or discharging the cleansing solution from the container for use in cleansing, the dilution ratio of the cleansing composition is preferably 1-fold or more and 100-fold or less, more preferably 2-fold or more and 50-fold or less, further more preferably 3-fold or more and 10-fold or less. The 1-fold dilution ratio means that the composition is not diluted with water.

When an apparel product is to be cleansed with the cleansing solution of the present invention, or in the case of a cleansing solution prepared by diluting a so-called high-concentration-type cleansing composition with water, the dilution ratio of the cleansing composition is preferably 100-fold or more, more preferably 150-fold or more, further more preferably 200-fold or more, from the viewpoint of excellent rinsability after cleansing, and preferably 5 000-fold or less, more preferably 4 000-fold or less, further more preferably 3 000-fold or less, from the viewpoint of exerting excellent cleansing performance. When an apparel product is to be cleansed with the cleansing solution of the present invention, or in the case of a cleansing solution prepared by diluting a so-called high-concentration-type cleansing composition with water, the dilution ratio of the cleansing composition is preferably 100-fold or more and 5 000-fold or less, more preferably 150-fold or more and 4 000-fold or less, further more preferably 200-fold or more and 3 000-fold or less.

The pH at 25°C of the cleansing solution of the present invention is preferably 2.5 or higher, more preferably 3 or higher, from the viewpoint of cleansing performance, and preferably 11 or lower, more preferably 10 or lower, further more preferably 9.5 or lower, from the viewpoint of suppressing damage on the surface to be cleansed. The pH at 25°C of the cleansing solution of the present invention is preferably 2.5 or higher and 11 or lower, more preferably 2.5 or higher and 10 or lower, further more preferably 3 or higher and 9.5 or lower.

A cleansing method using the cleansing composition of the present invention and water (hereinafter, also referred to as the cleansing method of the present invention) can cleanse a hard-surface article, an apparel product, or a body while exerting excellent cleansing performance without being influenced by the hardness or temperature of water for use in cleansing.

In the cleansing method of the present invention, the temperature of the water for use in cleansing can be 5°C or higher, may be 15°C or higher, and may also be 20°C or higher, and is usually 60°C or lower, may be 50°C or lower, and may

further be 40°C or lower.

The present invention is also highly useful for an aqueous solution of surfactant, including the following components (A) to (C):

(A) a glycolipid biosurfactant,
(B) an internal olefin sulfonate having 8 or more and 24 or less carbon atoms, wherein a content of an internal olefin sulfonate having a sulfonic acid group at the 2-position is 40% by mass or less, and
(C) water.

This aqueous solution of surfactant can be appropriately diluted or added to an agent containing a desired component, upon cleansing, and can make great contribution to stably exerting excellent cleansing performance while circumventing the formation of unnecessary deposits, without being influenced by the hardness or temperature of the water used.

The respective contents and mass ratios of the components (A) to (C) contained in the aqueous solution of surfactant of the present invention, and other components and their contents are the same as in the cleansing composition of the present invention.

The present invention can also provide a kit for cleansing, comprising:

an agent A including (A) a glycolipid biosurfactant; and
an agent B including (B) an internal olefin sulfonate having 8 or more and 24 or less carbon atoms, wherein a content of an internal olefin sulfonate having a sulfonic acid group at the 2-position is 40% by mass or less.

Specifically, the kit for cleansing of the present invention has a separate and independent agent A and agent B as members, and these parts are appropriately diluted with water while mixed, when used in cleansing. As a result, the respective parts can separately, independently, and stably contain the components (A) and (B) during preservation and before use and are capable of exerting excellent cleansing performance upon cleansing.

The agent A and the agent B can be diluted with water upon cleansing such that the component (A) and the component (B) satisfy the respective contents and mass ratios of the component (A) and the component (B) in the cleansing composition of the present invention. The kit may appropriately contain other components, as in the cleansing composition of the present invention.

In relation to the embodiments mentioned above, the present invention further discloses the following cleansing composition, cleansing solution, cleansing method, and use of the cleansing composition or the cleansing solution.

[1] A cleansing composition comprising the following components (A) and (B):

(A) a glycolipid biosurfactant, and
(B) an internal olefin sulfonate having 8 or more and 24 or less carbon atoms, wherein a content of an internal olefin sulfonate having a sulfonic acid group at the 2-position is 40% by mass or less.

[2] The cleansing composition according to [1], wherein the component (A) is preferably one or more selected from the group consisting of sophorolipid, rhamnolipid, trehalose lipid, and mannosyl alditol lipid, more preferably one or more selected from the group consisting of sophorolipid and rhamnolipid, further more preferably sophorolipid.

[3] The cleansing composition according to [1] or [2], wherein a content of the sophorolipid in the component (A) is preferably 50% by mass or more, more preferably 70% by mass or more, further more preferably 90% by mass or more, and may be 100% by mass.

[4] The cleansing composition according to any one of [1] to [3], wherein in the case of using sophorolipid as the component (A), a mass ratio of a content of a lactonic form (LSL) to a content of an acidic form (ASL) (LSL/ASL) in the sophorolipid is preferably 5 or less, more preferably 3 or less, and preferably 2 or more, or the sophorolipid may not contain LSL.

[5] The cleansing composition according to any one of [1] to [4], wherein the number of carbon atoms in the component (B) is preferably 12 or more, more preferably 16 or more, and 24 or less, preferably 22 or less, more preferably 20 or less, further more preferably 18 or less.

[6] The cleansing composition according to any one of [1] to [5], wherein the content of the internal olefin sulfonate having a sulfonic acid group at the 2-position is preferably 35% by mass or less, more preferably 30% by mass or less, further more preferably 28% by mass or less, and preferably 10% by mass or more, further more preferably 15% by mass or more, in the component (B).

[7] The cleansing composition according to any one of [1] to [6], wherein a mass ratio of a content of an internal olefin sulfonate (IO-1S) having 8 or more and 24 or less carbon atoms and having a sulfonic acid group at the 2- or higher and

4- or lower position to a content of an internal olefin sulfonate (IO-2S) having 8 or more and 24 or less carbon atoms and having a sulfonic acid group at the 5- or higher position ((IO-1S)/(IO-2S)) in the component (B) is preferably 0.50 or more, more preferably 0.60 or more, further more preferably 0.70 or more, and preferably 6.5 or less, more preferably 6.0 or less, further more preferably 5.5 or less, even more preferably 5.0 or less, even more preferably 4.5 or less, even more preferably 4.0 or less, even more preferably 3.5 or less, even more preferably 3.0 or less, even more preferably 2.5 or less, even more preferably 2.0 or less, even more preferably 1.5 or less.

[8] The cleansing composition according to any one of [1] to [7], wherein the content of the internal olefin sulfonate (IO-1S) having 8 or more and 24 or less carbon atoms and having a sulfonic acid group at the 2- or higher and 4- or lower position in the component (B) is preferably 90% by mass or less, more preferably 85% by mass or less, further more preferably 80% by mass or less, and preferably 30% by mass or more, more preferably 40% by mass or more.

[9] The cleansing composition according to any one of [1] to [8], wherein a mass ratio of an olefin (IO-1) having 8 or more and 24 or less carbon atoms and having a double bond at the 1- or higher and 3- or lower position to an olefin (IO-2) having 8 or more and 24 or less carbon atoms and having a double bond at the 5- or higher position ((IO-1)/(IO-2)) in an internal olefin having 8 or more and 24 or less carbon atoms for obtaining the component (B) is preferably 6.5 or less, more preferably 6.0 or less, further more preferably 5.5 or less, even more preferably 5.0 or less, even more preferably 4.5 or less, even more preferably 3.0 or less, even more preferably 2.5 or less, even more preferably 2.0 or less, even more preferably 1.5 or less, and preferably 0.50 or more, more preferably 0.55 or more, further more preferably 0.60 or more.

[10] The cleansing composition according to any one of [1] to [9], wherein a mass ratio of a content of the component (A) to a content of the component (B) ((A)/(B)) is preferably 0.0050 or more, more preferably 0.050 or more, further more preferably 0.10 or more, even more preferably 0.20 or more, and preferably 95.00 or less, more preferably 19.00 or less, further more preferably 9.00 or less, even more preferably 4.00 or less, even more preferably 2.00 or less, even more preferably 1.00 or less.

[11] The cleansing composition according to any one of [1] to [10], wherein a mass ratio of a content of the component (A) to a total content of the component (A) and the component (B) ((A)/{(A) + (B)}) is preferably 0.0050 or more, more preferably 0.010 or more, further more preferably 0.10 or more, even more preferably 0.20 or more, and preferably 0.99 or less, more preferably 0.95 or less, further more preferably 0.90 or less, further more preferably 0.80 or less, further more preferably 0.70 or less, further more preferably 0.60 or less, even more preferably 0.50 or less.

[12] The cleansing composition according to any one of [1] to [11], wherein the total content of the component (A) and the component (B) can be 0.15% by mass or more, is preferably 1.5% by mass or more, more preferably 2% by mass or more, further more preferably 3% by mass or more, and may be 55% by mass or less, may also be 50% by mass or less, or may further be 45% by mass or less, or the total content of the component (A) and the component (B) is preferably 100% by mass or less.

[13] The cleansing composition according to [12], wherein when the cleansing composition of the present invention is a low-concentration-type cleansing composition, the total content of the component (A) and the component (B) is preferably 0.15% by mass or more, more preferably 1.5% by mass or more, further more preferably 2% by mass or more, even more preferably 3% by mass or more, and preferably 25% by mass or less, more preferably 20% by mass or less, further more preferably 15% by mass or less, even more preferably 10% by mass or less.

[14] The cleansing composition according to [12], wherein when the cleansing composition of the present invention is a high-concentration-type cleansing composition, the total content of the component (A) and the component (B) is preferably 14% by mass or more, more preferably 16% by mass or more, further more preferably 18% by mass or more, even more preferably 20% by mass or more, and preferably 55% by mass or less, more preferably 50% by mass or less, further more preferably 45% by mass or less, even more preferably 40% by mass or less.

[15] The cleansing composition according to any one of [1] to [14], wherein the content of the component (A) can be 0.01% by mass or more, is preferably 0.04% by mass or more, more preferably 0.4% by mass or more, further more preferably 1% by mass or more, and may be 50% by mass or less, may also be 40% by mass or less, may further be 30% by mass or less, may further be 20% by mass or less, may further be 15% by mass or less, and may still further be 10% by mass or less.

[16] The cleansing composition according to [15], wherein when the cleansing composition of the present invention is a low-concentration-type cleansing composition, the content of the component (A) is preferably 0.01% by mass or more, more preferably 0.04% by mass or more, further more preferably 0.4% by mass or more, even more preferably 1% by mass or more, and preferably 10% by mass or less, more preferably 5% by mass or less, further more preferably 3% by mass or less, further more preferably 2.3% by mass or less, even more preferably 1.5% by mass or less.

[17] The cleansing composition according to [15], wherein when the cleansing composition of the present invention is a high-concentration-type cleansing composition, the content of the component (A) is preferably 1% by mass or more, more preferably 2% by mass or more, further more preferably 4% by mass or more, even more preferably 5% by mass or more, and preferably 50% by mass or less, more preferably 40% by mass or less, further more preferably 30% by mass or less, further more preferably 20% by mass or less, even more preferably 15% by mass or less, even more

preferably 10% by mass or less.

[18] The cleansing composition according to any one of [1] to [17], wherein the content of the component (B) can be 0.1% by mass or more, is preferably 1% by mass or more, more preferably 1.5% by mass or more, further more preferably 2.6% by mass or more, even more preferably 3.5% by mass or more, and may be 50% by mass or less, may also be 40% by mass or less, may further be 35% by mass or less, and may still further be 30% by mass or less.

[19] The cleansing composition according to [18], wherein when the cleansing composition of the present invention is a low-concentration-type cleansing composition, the content of the component (B) is preferably 0.1% by mass or more, more preferably 1% by mass or more, further more preferably 1.5% by mass or more, further more preferably 2.6% by mass or more, even more preferably 3.5% by mass or more, and preferably 20% by mass or less, more preferably 10% by mass or less, further more preferably 5% by mass or less, even more preferably 4% by mass or less.

[20] The cleansing composition according to [18], wherein when the cleansing composition of the present invention is a high-concentration-type cleansing composition, the content of the component (B) is preferably 1% by mass or more, more preferably 5% by mass or more, further more preferably 8% by mass or more, further more preferably 10% by mass or more, further more preferably 20% by mass or more, even more preferably 25% by mass or more, and preferably 50% by mass or less, more preferably 40% by mass or less, further more preferably 35% by mass or less, even more preferably 30% by mass or less.

[21] The cleansing composition according to any one of [1] to [20], wherein a water content of the component (C) may be 99% by mass or less and may be 30% by mass or more.

[22] The cleansing composition according to [21], wherein when the cleansing composition of the present invention is a low-concentration-type cleansing composition, the content of the component (C) is preferably 70% by mass or more, more preferably 75% by mass or more, further more preferably 80% by mass or more, even more preferably 85% by mass or more, and preferably 99% by mass or less, more preferably 98% by mass or less, further more preferably 97% by mass or less, even more preferably 95% by mass or less.

[23] The cleansing composition according to [21], wherein when the cleansing composition of the present invention is a high-concentration-type cleansing composition, the content of the component (C) is preferably 20% by mass or more, more preferably 30% by mass or more, further more preferably 35% by mass or more, even more preferably 40% by mass or more, and preferably 85% by mass or less, more preferably 80% by mass or less, further more preferably 75% by mass or less, even more preferably 70% by mass or less.

[24] The cleansing composition according to any one of [1] to [23], wherein a content of an additional surfactant other than the component (A) and the component (B) is preferably 10% by mass or less, more preferably 5% by mass or less, or the cleansing composition of the present invention may not contain the additional surfactant other than the component (A) and the component (B).

[25] The cleansing composition according to [24], wherein when the cleansing composition of the present invention is a low-concentration-type cleansing composition, the total content of the component (A) and the component (B) in 100% by mass as the total content of all the surfactants of the component (A), the component (B), and the additional surfactant other than the component (A) and the component (B) is preferably 10% by mass or more, more preferably 20% by mass or more, further more preferably 30% by mass or more, even more preferably 50% by mass or more, and preferably 100% by mass or less.

[26] The cleansing composition according to [24], wherein when the cleansing composition of the present invention is a high-concentration-type cleansing composition, the total content of the component (A) and the component (B) in 100% by mass as the total content of all the surfactants of the component (A), the component (B), and the additional surfactant other than the component (A) and the component (B) is preferably 10% by mass or more, more preferably 20% by mass or more, further more preferably 30% by mass or more, even more preferably 50% by mass or more, and preferably 100% by mass or less.

[27] The cleansing composition according to any one of [1] to [26], wherein a content of a chelating agent is preferably 10% by mass or less, more preferably 5% by mass or less, further more preferably 1% by mass or less, or the cleansing composition of the present invention may not contain the chelating agent.

[28] The cleansing composition according to any one of [1] to [27], wherein a content of a solvent other than the water is preferably 30% by mass or less, more preferably 20% by mass or less, further more preferably 10% by mass or less, or the cleansing composition of the present invention may not contain the solvent other than the water.

[29] The cleansing composition according to any one of [1] to [28], wherein pH at 25°C is preferably 3 or higher, more preferably 4 or higher, and preferably 13.5 or lower.

[30] A cleansing composition comprising the following components (A), (B), and (C):

(A) a glycolipid biosurfactant which is preferably one or more selected from the group consisting of sophorolipid, rhamnolipid, trehalose lipid, and mannosyl alditol lipid, more preferably one or more selected from the group consisting of sophorolipid and rhamnolipid, further more preferably sophorolipid,
(B) an internal olefin sulfonate wherein a content of an internal olefin sulfonate having a sulfonic acid group at the

2-position is 40% by mass or less, and the number of carbon atoms is preferably 12 or more, more preferably 16 or more, and 24 or less, preferably 22 or less, more preferably 20 or less, further more preferably 18 or less, and (C) water.

[31] A cleansing composition comprising the following components (A), (B), and (C):

(A) sophorolipid,
(B) an internal olefin sulfonate having 16 or more and 18 or less carbon atoms, wherein a content of an internal olefin sulfonate having a sulfonic acid group at the 2-position is 40% by mass or less, and
(C) water.

[32] A cleansing composition comprising the following components (A), (B), and (C):

(A) sophorolipid wherein a mass ratio of a content of a lactonic form (LSL) to a content of an acidic form (ASL) (LSL/ASL) is preferably 5 or less, more preferably 3 or less, preferably 2 or more, or sophorolipid which may not contain LSL,
(B) an internal olefin sulfonate having 16 or more and 18 or less carbon atoms, wherein a content of an internal olefin sulfonic acid having a sulfonic acid group at the 2-position is 40% by mass or less, and
(C) water.

[33] A cleansing composition comprising the following components (A), (B), and (C):

(A) sophorolipid wherein a mass ratio of a content of a lactonic form (LSL) to a content of an acidic form (ASL) (LSL/ASL) is preferably 5 or less,
(B) an internal olefin sulfonate having 16 or more and 18 or less carbon atoms, wherein a content of an internal olefin sulfonate having a sulfonic acid group at the 2-position is 40% by mass or less, and
(C) water.

[34] A cleansing composition comprising the following components (A), (B), and (C):

(A) sophorolipid wherein a mass ratio of a content of a lactonic form (LSL) to a content of an acidic form (ASL) (LSL/ASL) is preferably 5 or less,
(B) an internal olefin sulfonate having 16 or more and 18 or less carbon atoms, wherein a content of an internal olefin sulfonate having a sulfonic acid group at the 2-position is 40% by mass or less, and
(C) water, wherein
a mass ratio of a content of the component (A) to a total content of the component (A) and the component (B) ((A)/{(A) + (B)}) is preferably 0.0050 or more, more preferably 0.010 or more, further more preferably 0.10 or more, even more preferably 0.20 or more, and preferably 0.99 or less, more preferably 0.95 or less, further more preferably 0.90 or less, further more preferably 0.80 or less, further more preferably 0.70 or less, further more preferably 0.60 or less, even more preferably 0.50 or less.

[35] A cleansing composition comprising the following components (A), (B), and (C):

(A) sophorolipid wherein a mass ratio of a content of a lactonic form (LSL) to a content of an acidic form (ASL) (LSL/ASL) is preferably 5 or less,
(B) an internal olefin sulfonate having 16 or more and 18 or less carbon atoms, wherein a content of an internal olefin sulfonate having a sulfonic acid group at the 2-position is 40% by mass or less, and
(C) water, wherein
a mass ratio of a content of the component (A) to a total content of the component (A) and the component (B) ((A)/{(A) + (B)}) is 0.20 or more and 0.80 or less.

[36] A cleansing composition comprising the following components (A), (B), and (C):

(A) sophorolipid wherein a mass ratio of a content of a lactonic form (LSL) to a content of an acidic form (ASL) (LSL/ASL) is preferably 5 or less,
(B) an internal olefin sulfonate having 16 or more and 18 or less carbon atoms, wherein a content of an internal olefin sulfonate having a sulfonic acid group at the 2-position is 40% by mass or less, and
(C) water, wherein

a mass ratio of a content of the component (A) to a total content of the component (A) and the component (B) ((A)/{(A) + (B)}) is 0.20 or more and 0.70 or less.

[37] A cleansing composition comprising the following components (A), (B), and (C):

(A) sophorolipid wherein a mass ratio of a content of a lactonic form (LSL) to a content of an acidic form (ASL) (LSL/ASL) is preferably 5 or less,
(B) an internal olefin sulfonate having 16 or more and 18 or less carbon atoms, wherein a content of an internal olefin sulfonate having a sulfonic acid group at the 2-position is 40% by mass or less, and
(C) water, wherein
a mass ratio of a content of the component (A) to a total content of the component (A) and the component (B) ((A)/{(A) + (B)}) is 0.20 or more and 0.50 or less.

[38] A cleansing composition comprising the following components (A), (B), and (C):

(A) sophorolipid wherein a mass ratio of a content of a lactonic form (LSL) to a content of an acidic form (ASL) (LSL/ASL) is preferably 3 or less,
(B) an internal olefin sulfonate having 16 or more and 18 or less carbon atoms, wherein a content of an internal olefin sulfonate having a sulfonic acid group at the 2-position is 40% by mass or less, and
(C) water, wherein
a mass ratio of a content of the component (A) to a total content of the component (A) and the component (B) ((A)/{(A) + (B)}) is 0.20 or more and 0.80 or less.

[39] A cleansing composition comprising the following components (A), (B), and (C):

(A) sophorolipid wherein a mass ratio of a content of a lactonic form (LSL) to a content of an acidic form (ASL) (LSL/ASL) is preferably 3 or less,
(B) an internal olefin sulfonate having 16 or more and 18 or less carbon atoms, wherein a content of an internal olefin sulfonate having a sulfonic acid group at the 2-position is 40% by mass or less, and
(C) water, wherein
a mass ratio of a content of the component (A) to a total content of the component (A) and the component (B) ((A)/{(A) + (B)}) is 0.20 or more and 0.70 or less.

[40] A cleansing composition comprising the following components (A), (B), and (C):

(A) sophorolipid wherein a mass ratio of a content of a lactonic form (LSL) to a content of an acidic form (ASL) (LSL/ASL) is preferably 3 or less,
(B) an internal olefin sulfonate having 16 or more and 18 or less carbon atoms, wherein a content of an internal olefin sulfonate having a sulfonic acid group at the 2-position is 40% by mass or less, and
(C) water, wherein
a mass ratio of a content of the component (A) to a total content of the component (A) and the component (B) ((A)/{(A) + (B)}) is 0.20 or more and 0.50 or less.

[41] A cleansing composition comprising the following components (A), (B), and (C):

(A) sophorolipid wherein a mass ratio of a content of a lactonic form (LSL) to a content of an acidic form (ASL) (LSL/ASL) is preferably 5 or less,
(B) an internal olefin sulfonate having 16 carbon atoms, wherein a content of an internal olefin sulfonic acid having a sulfonate group at the 2-position is 40% by mass or less, and
(C) water, wherein
a mass ratio of a content of the component (A) to a total content of the component (A) and the component (B) ((A)/{(A) + (B)}) is 0.20 or more and 0.80 or less.

[42] A cleansing composition comprising the following components (A), (B), and (C):

(A) sophorolipid wherein a mass ratio of a content of a lactonic form (LSL) to a content of an acidic form (ASL) (LSL/ASL) is preferably 5 or less,
(B) an internal olefin sulfonate having 16 carbon atoms, wherein a content of an internal olefin sulfonate having a

sulfonic acid group at the 2-position is 40% by mass or less, and
(C) water, wherein
a mass ratio of a content of the component (A) to a total content of the component (A) and the component (B) ((A)/{(A) + (B)}) is 0.20 or more and 0.70 or less.

[43] A cleansing composition comprising the following components (A), (B), and (C):

(A) sophorolipid wherein a mass ratio of a content of a lactonic form (LSL) to a content of an acidic form (ASL) (LSL/ASL) is preferably 5 or less,
(B) an internal olefin sulfonate having 16 carbon atoms, wherein a content of an internal olefin sulfonic acid having a sulfonate group at the 2-position is 40% by mass or less, and
(C) water, wherein
a mass ratio of a content of the component (A) to a total content of the component (A) and the component (B) ((A)/{(A) + (B)}) is 0.20 or more and 0.50 or less.

[44] A cleansing composition for apparel, comprising the following components (A), (B), and (C):

(A) sophorolipid wherein a mass ratio of a content of a lactonic form (LSL) to a content of an acidic form (ASL) (LSL/ASL) is preferably 5 or less,
(B) an internal olefin sulfonate having 16 carbon atoms, wherein a content of an internal olefin sulfonic acid having a sulfonate group at the 2-position is 40% by mass or less, and
(C) water, wherein
a mass ratio of a content of the component (A) to a total content of the component (A) and the component (B) ((A)/{(A) + (B)}) is 0.20 or more and 0.50 or less.

[45] A cleansing composition for a hard-surface article or a body, comprising the following components (A), (B), and (C):

(A) sophorolipid wherein a mass ratio of a content of a lactonic form (LSL) to a content of an acidic form (ASL) (LSL/ASL) is preferably 5 or less,
(B) an internal olefin sulfonate having 16 or more and 18 or less carbon atoms, wherein a content of an internal olefin sulfonate having a sulfonic acid group at the 2-position is 40% by mass or less, and
(C) water, wherein
a mass ratio of a content of the component (A) to a total content of the component (A) and the component (B) ((A)/{(A) + (B)}) is 0.20 or more and 0.80 or less.

[46] A cleansing solution prepared by diluting the cleansing composition according to any one of [1] to [45] with water, wherein a hardness of the water for use in dilution can be 0.1 °DH or more, may be 1 °DH or more, may also be 2 °DH or more, may further be 3 °DH or more, and is usually 30 °DH or less, may be 25 °DH or less, may further be 20 °DH or less.
[47] The cleansing solution according to [45], wherein a total content of the component (A) and the component (B) is preferably $5 \times 10^{-5}$% by mass or more, more preferably $1 \times 10^{-3}$% by mass or more, further more preferably $5 \times 10^{-3}$% by mass or more, even more preferably $1.5 \times 10^{-2}$% by mass or more, and may be 10% by mass or less.
[48] The cleansing solution according to [46] or [47], wherein a dilution ratio of the cleansing composition according to any one of [1] to [45] may be 1.5-fold or more, may be 2-fold or more, or may be 3-fold or more, and may be 10 000-fold or less, may be 5 000-fold or less, or may be 2 000-fold or less.
[49] The cleansing solution according to [48], wherein when the cleansing solution of the present invention is a cleansing solution prepared by diluting a low-concentration-type cleansing composition with water, the dilution ratio of the cleansing composition is preferably 1-fold or more, more preferably 2-fold or more, further more preferably 3-fold or more, and preferably 100-fold or less, more preferably 50-fold or less, further more preferably 10-fold or less.
[50] The cleansing solution according to [48], wherein when the cleansing solution of the present invention is a cleansing solution prepared by diluting a high-concentration-type cleansing composition with water, the dilution ratio of the cleansing composition is preferably 100-fold or more, more preferably 150-fold or more, further more preferably 200-fold or more, and preferably 5 000-fold or less, more preferably 4 000-fold or less, further more preferably 3 000-fold or less.
[51] The cleansing solution according to any one of [46] to [50], wherein pH at 25°C is preferably 2.5 or higher, more preferably 3 or higher, and preferably 11 or lower, more preferably 10 or lower, further more preferably 9.5 or lower.
[52] A cleansing method using the cleansing composition according to any one of [1] to [45] and water, wherein a

hardness of the water is 30 °DH or less.

[53] A cleansing method using the cleansing solution according to any one of [46] to [51] and water, wherein a hardness of the water is 30 °DH or less.

[54] The cleansing method according to [52] or [53], wherein a temperature of the water is 5°C or higher and 60°C or lower.

[55] Use of the cleansing composition according to any one of [1] to [45] for a hard-surface article or a body to be cleansed, or for filling a container such as a spray container or a pump foamer with the cleansing composition, and spraying or discharging the cleansing composition from the container for use in cleansing.

[56] Use of the cleansing composition according to any one of [1] to [45] for an apparel product to be cleansed.

[57] Use of the cleansing solution according to any one of [46] to [51] for a hard-surface article or a body to be cleansed, or for filling a container such as a spray container or a pump foamer with the cleansing solution, and spraying or discharging the cleansing solution from the container for use in cleansing.

[58] Use of the cleansing solution according to any one of [46] to [51] for an apparel product to be cleansed.

Examples

Hereinafter, the present invention will be specifically described with reference to Examples. The content of each component is indicated by % by mass unless otherwise specified in tables.

Methods for measuring various physical properties are as follows.

[Methods for measuring various physical properties]

(i) Measurement of mass ratio of contents of lactonic form(LSL), acidic form(ASL), and fatty acid (LSL:ASL:fatty acid) contained in sophorolipid sample

High-performance liquid chromatography (HPLC) was used in measurement. Specifically, LSL, ASL, and fatty acid were separated by HPLC and each applied to a corona charged aerosol detector (CAD) for identification. Their respective contents were determined from the resulting HPLC-CAD peak areas. The fatty acid here corresponds to oleic acid.

The apparatuses and conditions used in measurement were as follows: HPLC apparatus "Chromaster" (manufactured by Hitachi High-Tech Corp.), column "L-column ODS(R) "(4.6 $\times$ 150 mm, particle size: 5 $\mu$m, manufactured by Chemical Evaluation and Research Institute, Japan), eluent A (water supplemented with 10 mM ammonium acetate), eluent B (acetonitrile/water = 95/5 (v/v) solution supplemented with 10 mM ammonium acetate), gradient (0 to 5 min (A/B = 60/40) $\rightarrow$ 10 min (50/50) $\rightarrow$ 30 min (40/60), 50 to 60 min (0/100), CAD apparatus "Corona CAD" (manufactured by ESA Biosciences, Inc.), column temperature (40°C), flow rate (0.5 mL/min), injection volume (5 $\mu$L)

(ii) Method for measuring double bond position of starting material olefin

A double bond position of a starting material olefin was measured by gas chromatography (hereinafter, abbreviated to GC). Specifically, the starting material olefin was converted to a dithio derivative through reaction with dimethyl disulfide, followed by the separation of each component by GC. The double bond position of the starting material olefin was determined from each peak area.

The apparatuses and analysis conditions used in measurement were as follows: GC apparatus (trade name: HP6890, manufactured by Hewlett-Packard Company), column (trade name: Ultra-Alloy-1HT capillary column 30 m $\times$ 250 $\mu$m $\times$ 0.15 $\mu$m, manufactured by Frontier Laboratories Ltd.), detector (hydrogen flame ionization detector (FID)), injection temperature 300°C, detector temperature 350°C, He flow rate 4.6 mL/min

(iii) Method for measuring content appropriate for sulfonic acid group bond position of sodium internal olefin sulfonate

Each sodium internal olefin sulfonate content appropriate for a sulfonic acid group bond position was measured as to sodium internal olefin sulfonate with a sulfonic acid group bonded thereto using a high-performance liquid chromatography/mass spectrometer (HPLC-MS). Specifically, hydroxy forms with a sulfonic acid group bonded thereto were separated by high-performance liquid chromatography (HPLC) and each applied to a mass spectrometer (MS) for identification. Their respective contents were determined from the resulting HPLC-MS peak areas.

The apparatuses and conditions used in measurement were as follows: HPLC apparatus "LD20ASXR" (manufactured by Shimadzu Corp.), column "ODS Hypersil(R)" (4.6 $\times$ 250 mm, particle size: 3 $\mu$m, manufactured by Thermo Fisher Scientific Inc.), sample preparation (1 000-fold dilution with methanol), eluent A (water supplemented with 10 mM ammonium acetate), eluent B (methacrylonitrile/water = 95/5 (v/v) solution supplemented with 10 mM ammonium

acetate), gradient (0 min (A/B = 60/40) → 15.1 to 20 min (30/70) → 20.1 to 30 min (60/40), MS apparatus "LCMS-2020" (manufactured by Shimadzu Corp.), ESI detection (anion detection m/z: 321.10 (component (A) having 16 or 18 carbon atoms)), column temperature (40°C), flow rate (0.5 mL/min), injection volume (5 μL)

(iv) Method for measuring hydroxy form/olefin form mass ratio

A hydroxy form/olefin form mass ratio of sodium internal olefin sulfonate was measured by HPLC-MS. Specifically, a hydroxy form and an olefin form were separated by HPLC and each applied to MS for identification. Their respective proportions were determined from the resulting HPLC-MS peak areas.

The apparatuses and conditions used in measurement were as follows: HPLC apparatus (trade name: Agilent 1100, manufactured by Agilent Technologies, Inc.), column (trade name: L-column ODS 4.6 × 150 mm, manufactured by Chemical Evaluation and Research Institute, Japan), sample preparation (1 000-fold dilution with methanol), eluent A (water supplemented with 10 mM ammonium acetate), eluent B (methanol supplemented with 10 mM ammonium acetate), gradient (0 min (A/B = 30/70%) → 10 min (30/70%) - 55 min (0/100%) → 65 min (0/100%) → 66 min (30/70%) - 75 min (30/70%)), MS apparatus (trade name: Agilent 1100MS SL (G1946D)), MS detection (anion detection m/z 60-1600, UV 240 nm)

(v) Method for measuring content of starting material olefin

A content of an unreacted starting material olefin in sodium internal olefin sulfonate was measured by GC. Specifically, ethanol and petroleum ether were added to an aqueous sodium internal olefin sulfonate solution, followed by extraction to obtain an olefin in a petroleum ether phase. The starting material olefin was quantified from the resulting GC peak area.

The apparatuses and analysis conditions used in measurement were as follows: GC apparatus (trade name: Agilent 6850, manufactured by Agilent Technologies, Inc.), column (trade name: Ultra-Alloy-1HT capillary column 15 m × 250 μm × 0.15 μm, manufactured by Frontier Laboratories Ltd.), detector (hydrogen flame ionization detector (FID)), injection temperature 300°C, detector temperature 350°C, He flow rate 3.8 mL/min

(vi) Method for measuring content of inorganic compound

A content of an inorganic compound was measured by potentiometric titration or neutralizing titration. Specifically, a content of $Na_2SO_4$ was quantified by determining silver sulfate ($SO_4^{2-}$) by potentiometric titration. A content of NaOH was quantified by neutralizing titration with dilute hydrochloric acid.

[Production Example A1: production of sophorolipid A1]

Sophorolipid A1 was produced in accordance with the following procedures 1.) to 4.).

1.) Plate culture

A *Candida bombicola* NBRC10243 strain was used, and one loopful of an inoculant was seeded to a Petri dish with an agar medium containing 1% glucose, 1% yeast extracts, 1% tryptone, and 1.5% agar, and cultured at a temperature of 30°C for 2 days.

2.) Preculture

100 mL of a culture solution containing 1% glucose, 1% yeast extracts, and 1% tryptone was placed in a Sakaguchi flask and sterilized at a high temperature of 121°C for 20 minutes. After cooling, one loopful of the inoculant was seeded thereto from the plate and cultured by stirring for 2 days under conditions involving a temperature of 30°C and a stirring rotation speed of 120 r/min.

3.) Main culture

To 10 kg rapeseed oil, 10 kg of distilled water was added, and Lipase AY 30SD (manufactured by Amano Enzyme Inc.) was added at 0.1%. The rapeseed oil prepared by dilution by the method mentioned above was stirred for 24 hours under a condition of 30°C and then left standing for 1 hour, and a separated upper phase was recovered. 10 kg of distilled water was added to the recovered upper phase, which was then stirred and dispersed, and then left standing for 1 hour while warmed at 30°C again, and a separated and recovered upper phase was used as rapeseed oil-derived fatty acid. A

culture solution containing 5% rapeseed oil-derived fatty acid, 12.5% glucose, 2% yeast extracts, and 0.1% urea was prepared and brought to 15 L. The culture solution was sterilized in a 30 L (total capacity) jar fermenter, to which 300 mL of the preculture solution was then added for inoculation and cultured by stirring for 96 hours from the start of culture under conditions involving a temperature of 30°C, a stirring rotation speed of 300 r/min, and an aeration rate (aeration volume) of 7.5 L/min (0.5 vvm).

An aeration oxygen concentration was set to 21% over the whole culture period.

4.) Collection of sophorolipid

The obtained culture solution was left standing, and formed precipitates were collected. Distilled water warmed at 75°C was added to the collected precipitates, which were then stirred and dispersed, and then left standing for 30 minutes to recover precipitates again. This washing operation was repeated three times to obtain sophorolipid A1.

The obtained sophorolipid A1 had a mass ratio (LSL:ASL:fatty acid) of 76.0:18.9:5.1.

[Production Example A2: production of sophorolipid A2]

100 mL of the sophorolipid A1 obtained in Production Example 1 was collected, placed in a 300 mL flask, and warmed to 50°C in a water bath with stirring using a stirrer. To a lactonic form of sophorolipid in the sophorolipid sample thus warmed, 6 equivalents (29 g) of a 50% aqueous potassium hydroxide solution was added, and the mixture was stirred for 30 minutes to obtain sophorolipid A2.

The obtained sophorolipid A2 had a mass ratio (LSL:ASL:fatty acid) of 0:94.9:5.1.

Sophorolipid A3 was obtained in the same manner as in Production Example A2 except that 1 equivalent (4.8 g) of a 50% aqueous potassium hydroxide solution was added to the lactonic form of sophorolipid in the sophorolipid sample.

The obtained sophorolipid A3 had a mass ratio (LSL:ASL:fatty acid) of 69.8:25.1:5.1.

[Production Example b1: production of starting material olefin b1 having 16 carbon atoms]

A flask with a stirring apparatus was charged with 7 000 g (28.9 mol) of 1-hexadecanol (product name: KALKOL 6098, manufactured by Kao Corp.) and 350 g (5% by mass based on the starting material alcohol) of a solid acid catalyst γ-alumina (manufactured by STREM Chemicals, Inc.), which were then reacted for 8 hours with stirring while nitrogen (7 000 mL/min) was circulated in the system at 280°C. After the completion of reaction, an alcohol conversion rate was 100%. The obtained crude alkene internal olefin was transferred to a flask for distillation and distilled at 136 to 160°C/4.0 mmHg to obtain a starting material olefin b1 having 16 carbon atoms with an olefin purity of 100%.

Table 1 shows the double bond distribution of the obtained starting material olefin b1.

[Production Example b2: production of starting material olefin b2 having 18 carbon atoms]

A starting material olefin b2 having 18 carbon atoms with an olefin purity of 100% was obtained in the same manner as in Production Example b1 except that 1-octadecanol (KALKOL 8098, manufactured by Kao Corp.) was used.

Table 1 shows the double bond distribution of the obtained starting material olefin b2.

[Table 1]

| Starting material olefin | b1 | b2 |
|---|---|---|
| The number of carbon atoms | 16 | 18 |

(continued)

| Starting material olefin | | b1 | b2 |
|---|---|---|---|
| Double bond distribution in starting material olefin (% by mass) | 1-position | 1.9 | 1.5 |
| | 2-position | 21.0 | 26.0 |
| | 3-position | 18.2 | 21.3 |
| | 4-position | 18.7 | 17.7 |
| | 5-position | 14.8 | 11.8 |
| | 6-position | 12.2 | 8.4 |
| | 7-position | 6.6 | 5.9 |
| | 8-position | 6.6 | 3.7 |
| | 9-position | - | 3.7 |
| | Total | 100.0 | 100.0 |
| Average double bond position (DBP) | | 4.2 | 4.0 |
| (IO-1)/(IO-2) | | 1.02 | 1.45 |

[Production Example B1: production of sodium internal olefin sulfonate B1 having 16 carbon atoms]

The starting material olefin b1 obtained in Production Example b1 was placed in a thin-film sulfonation reactor having an outside jacket, and sulfonation reaction was performed using sulfur trioxide gas under conditions involving injecting cooling water of 10°C to the outside jacket of the reactor. For the sulfonation reaction, an $SO_3$/internal olefin molar ratio was set to 1.01. The obtained sulfonation product was mixed with an alkaline aqueous solution prepared with a 1.04-fold molar amount of sodium hydroxide (alkali agent) based on a theoretical acid value, and neutralized at 30°C for 1 hour by a continuous method. The obtained neutralization product was hydrolyzed by heating at 170°C for 1 hour in an autoclave to obtain sodium internal olefin sulfonate B1 having 16 carbon atoms. The obtained sodium internal olefin sulfonate B1 having 16 carbon atoms contained the starting material olefin at a content of 0.4% by mass and contained 0.39% by mass of an inorganic compound.

Table 2 shows each physical property value of the obtained sodium internal olefin sulfonate B1.

[Production Example B2: production of sodium internal olefin sulfonate B2 having 18 carbon atoms]

Sodium internal olefin sulfonate B2 having 18 carbon atoms was obtained in the same manner as in Production Example B1 except that the starting material olefin b2 obtained in Production Example b2 was used as the starting material olefin. The obtained sodium internal olefin sulfonate B2 having 18 carbon atoms contained the starting material olefin at a content of the GC detection limit or less (100 ppm or less) and contained 0.2% by mass of an inorganic compound.

Table 2 shows each physical property value of the obtained sodium internal olefin sulfonate B2.

[Production Example B3: production of potassium internal olefin sulfonate B3 having 16 carbon atoms]

Potassium internal olefin sulfonate B3 having 16 carbon atoms was obtained in the same manner as in Production Example B1 except that an alkaline aqueous solution prepared with potassium hydroxide was used as the alkali agent. The obtained potassium internal olefin sulfonate B3 having 16 carbon atoms contained the starting material olefin at a content of 0.4% by mass and contained 0.39% by mass of an inorganic compound.

Table 2 shows each physical property value of the obtained potassium internal olefin sulfonate B3.

[Production Example B4: production of potassium internal olefin sulfonate B4 having 18 carbon atoms]

Potassium internal olefin sulfonate B4 having 18 carbon atoms was obtained in the same manner as in Production Example B2 except that an alkaline aqueous solution prepared with potassium hydroxide was used as the alkali agent. The obtained potassium internal olefin sulfonate B4 having 18 carbon atoms contained the starting material olefin at a content of the GC detection limit or less (100 ppm or less) and contained 0.2% by mass of an inorganic compound.

Table 2 shows each physical property value of the obtained potassium internal olefin sulfonate B4.

[Table 2]

| | | Sodium internal olefin sulfonate | | Potassium internal olefin sulfonate | |
|---|---|---|---|---|---|
| | | B1 | B2 | B3 | B4 |
| Starting material olefin | | b1 | b2 | b1 | b2 |
| Distribution of sulfonic acid group (% by mass) | 1-position | 1.7 | 1.4 | 1.7 | 1.4 |
| | 2-position | 17.5 | 20.7 | 17.5 | 20.7 |
| | 3-position | 15.6 | 17.4 | 15.6 | 17.4 |
| | 4-position | 20.3 | 21.0 | 20.3 | 21.0 |
| | 5 to 9-position | 44.9 | 39.5 | 44.9 | 39.5 |
| | Total | 100.0 | 100.0 | 100.0 | 100.0 |
| Hydroxy form | | 83.9 | 84.5 | 83.9 | 84.5 |
| Olefin form | | 16.1 | 15.5 | 16.1 | 15.5 |
| (IO-1S)/(IO-2S) | | 0.81 | 0.65 | 0.81 | 0.65 |

[Examples 1-1 to 1-4 and Comparative Examples 1-1 to 1-5]

Each cleansing composition having the compositional profile shown in Table 3 was prepared by a routine method. Specifically, the component (A) or the component (A'), the component (B), and the component (C) (ion exchange water) were weighed into a glass beaker, warmed to 60°C, mixed, and cooled to room temperature.
Subsequently, the obtained cleansing composition was used in each measurement and evaluation for the cleansing composition in accordance with the methods described below.
Model 4 000 °DH-hardness water was prepared by dissolving 84 g of calcium chloride dihydrate (manufactured by FUJIFILM Wako Pure Chemical Corp.) and 29 g of magnesium chloride hexahydrate (manufactured by FUJIFILM Wako Pure Chemical Corp.) in ion exchange water added so as to give 1 L (calcium ion: 0.57 mol/L, magnesium ion: 0.14 mol/L).
Subsequently, 2.6 mL of the obtained cleansing composition, 2.25 mL of the model 4 000 °DH water, and ion exchange water were mixed such that the amount of a liquid was 600 mL to prepare a cleansing solution (total content of the component (A) and the component (B): 0.15% by mass, hardness: 15 °DH). The pH of the cleansing solution was appropriately adjusted to the value shown in Table 3 using 1.0 mol/L hydrochloric acid (manufactured by FUJIFILM Wako Pure Chemical Corp.) and a 0.5 mol/L aqueous potassium hydroxide solution (manufactured by FUJIFILM Wako Pure Chemical Corp.).
Subsequently, the obtained cleansing solution was used in each measurement and evaluation for the cleansing solution in accordance with the methods described below.
The results are shown in Table 3.

<<pH at 25°C of cleansing composition>>

pH was measured at 25°C using a pH electrode (manufactured by HORIBA, Ltd., model number F-22).

<<Evaluation of fluidity at 25°C of cleansing composition>>

In an environment with room temperature adjusted to 25°C, 5 mL of the obtained cleansing composition was collected and housed in a glass screw tube. Subsequently, appearance was visually confirmed and evaluated in accordance with the following criteria.
X: When the screw tube was overturned, the cleansing composition flowed in accordance with the movement, was able to be easily taken out thereof in a uniform state, and thus found excellent in fluidity.
Z: In the screw tube, the cleansing composition underwent separation, or a highly viscous phase was unevenly distributed, and the cleansing composition was difficult to uniformly take out thereof and thus found inferior in fluidity.

<<Evaluation of colorless transparency at 25°C of cleansing composition>>

The appearance of the cleansing composition housed in a glass screw tube in the same manner as in the

evaluation of fluidity was visually confirmed and evaluated in accordance with the following criteria.

A: The cleansing composition was colorless and transparent.
B: The cleansing composition was pale yellow, but transparent.
C: The cleansing composition was dark yellow, but transparent without practical problems.
D: The cleansing composition was transparent, but brown with practical problems.
E: The cleansing composition was brown and opaque with practical problems.

<<pH at 25°C of cleansing solution>>

pH was measured at 25°C using a pH electrode (manufactured by HORIBA, Ltd., model number F-22).

<<Evaluation of cleansing performance of cleansing solution for artificial sebum-contaminated cloth to be cleansed>>

1.) Preparation of model artificial sebum-contaminated cloth

An artificial sebum liquid as model sebum (WFK 09D Synthetic sebum, BEY formulation, manufactured by wfk - Testgewebe GmbH) was uniformly dissolved at 60°C. Then, Sudan III (manufactured by FUJIFILM Wako Pure Chemical Corp.) was added at 0.1 wt% thereto, and the mixture was stirred until uniform using a stirrer in a water bath of 60°C. 0.1 mL of the resultant was added dropwise to polyester/cotton blend cloth ((broadcloth, supplied by Yagashira Shoten (Komatsu 4-11-15, Higashi Yodogawa, Osaka city, Osaka, Japan)) cut into 6 cm square, and the cloth was left standing for 3 hours in a thermostat bath of 55°C and uniformly spread over white cloth. Then, the cloth was left standing at 25°C for 2 hours to prepare model artificial sebum-contaminated cloth.

2.) Evaluation of cleansing performance

Cleansing performance was evaluated using Tergotometer (Ueshima, MS-8212) as a cleansing device. The Tergotometer is a rotary device which performs cleansing, and is a device generally used as a model cleansing device for a fully automatic washing machine for home use, a drum-type fully automatic washing machine, a pulsator-type fully automatic washing machine for home use, or an agitator-type fully automatic washing machine for home use. The Tergotometer is, in particular, a model cleansing device corresponding to a pulsator-type fully automatic washing machine for home use or an agitator-type fully automatic washing machine for home use.

Four pieces of the model artificial sebum-contaminated cloth prepared in the procedure 1.) were cleansed at 85 rpm for 10 minutes using the Tergotometer. The cloth thus cleansed was rinsed with tap water (20°C) for 3 minutes. Refractive indexes at 550 nm in the uncontaminated original cloth and before and after cleansing were measured with a colorimeter (manufactured by Nippon Denshoku Industries Co., Ltd., Z-300A). A cleansing rate (%) was calculated according to the following expression (x), and an average value of the four pieces of the model artificial sebum-contaminated cloth was determined and used as an index of evaluation.

$$\text{Cleansing rate (\%)} = 100 \times [(\text{Refractive index after cleansing} - \text{Refractive index before cleansing})/(\text{Refractive index of the original cloth} - \text{Refractive index before cleansing})] \quad (x)$$

The temperature of the cleansing solution was adjusted to 25°C or 40°C, and the cleansing performance was evaluated at each of the temperatures.

[Table 3]

| | | Example 1-1 | Example 1-2 | Example 1-3 | Example 1-4 | Comparative Example 1-1 | Comparative Example 1-2 | Comparative Example 1-3 | Comparative Example 1-4 | Comparative Example 1-5 |
|---|---|---|---|---|---|---|---|---|---|---|
| (A) | Sophorolipid A2 | 26.20 | 20.00 | 14.00 | 8.80 | 35.00 | | | | |
| (A') | Sodium N-dodecanoyl sarcosinate*1 | | | | | | | 17.50 | | |
| | Sodium cocoyl glutamate*2 | | | | | | | | 17.50 | |
| | Sodium polyoxyethylene lauryl ether acetate*3 | | | | | | | | | 17.50 |
| (B) | Potassium internal olefin sulfonate B3 | 8.80 | 15.00 | 21.00 | 26.20 | | 35.00 | 17.50 | 17.50 | 17.50 |
| (C) | Ion exchange water | Balance | Balance | Balance | Balance | Balance | Balance | Balance | Balance | Balance |
| | Total | 100.00 | 100.00 | 100.00 | 100.00 | 100.00 | 100.00 | 100.00 | 100.00 | 100.00 |
| | (A) + (B) (or (A) + (B)) | 35.00 | 35.00 | 35.00 | 35.00 | 35.00 | 35.00 | 35.00 | 35.00 | 35.00 |
| | (A)/(B) (or (A')/(B)) | 3.00 | 1.33 | 0.67 | 0.33 | - | - | 1.00 | 1.00 | 1.00 |
| | (A)/{(A) + (B)} (or (A')/{(A') + (B)}) | 0.75 | 0.57 | 0.40 | 0.25 | 1.00 | 0.00 | 0.50 | 0.50 | 0.50 |
| Cleansing composition | pH at 25°C | 13.4 | 13.6 | 13.7 | 13.7 | 13.8 | 9.5 | 5.9 | 8.5 | 5.8 |
| | Fluidity at 25°C | X | X | X | X | X | Z | Z | Z | Z |
| | Colorless transparency at 25°C | C | C | B | A | D | A | A | A | A |

(continued)

| | | Example 1-1 | Example 1-2 | Example 1-3 | Example 1-4 | Comparative Example 1-1 | Comparative Example 1-2 | Comparative Example 1-3 | Comparative Example 1-4 | Comparative Example 1-5 |
|---|---|---|---|---|---|---|---|---|---|---|
| Cleansing solution | pH at 25°C | 8.5 | 8.5 | 8.5 | 8.5 | 8.5 | 8.5 | 8.5 | 8.5 | 8.5 |
| | Cleansing performance at temperature of 25°C (%) | 35.7 | 31.6 | 31.0 | 28.2 | 26.6 | 19.9 | 3.6 | 12.8 | 24.3 |
| | Cleansing performance at temperature of 40°C (%) | 54.0 | 47.2 | 49.0 | 48.0 | 51.3 | 41.5 | 11.8 | 12.8 | 36.5 |

*1: manufactured by FUJIFILM Wako Pure Chemical Corp.
*2: AMISOFT CS-11(F), manufactured by Ajinomoto Co., Inc.
*3: KAO AKYPO RLM-4, manufactured by Kao Corp.

[Examples 2-1 to 2-4 and Comparative Examples 2-1 and 2-2]

Each cleansing composition was prepared in the same manner as in Example 1-1 in accordance with the compositional profile shown in Table 4.
Subsequently, 2.6 mL of the obtained cleansing composition, 0.6 mL of the model 4 000 °DH water, and ion exchange water were mixed such that the amount of a liquid was 600 mL to prepare a cleansing solution I (total content of the component (A) and the component (B): 0.15% by mass, hardness: 4 °DH). A cleansing solution II (total content of the component (A) and the component (B): 0.02% by mass, hardness: 4 °DH) was prepared in the same manner as in the cleansing solution I except that 0.3 mL of the cleansing composition was used. The pH of the cleansing solution was appropriately adjusted to the value shown in Table 4 using 1.0 mol/L hydrochloric acid (manufactured by FUJIFILM Wako Pure Chemical Corp.) and a 0.5 mol/L aqueous potassium hydroxide solution (manufactured by FUJIFILM Wako Pure Chemical Corp.).
Subsequently, each of the obtained cleansing solutions (temperature: 25°C) was used in evaluation for the cleansing solution in the same manner as in Example 1-1.
The results are shown in Table 4.

[Table 4]

| | | Example 2-1 | Example 2-2 | Example 2-3 | Example 2-4 | Comparative Example 2-1 | Comparative Example 2-2 |
|---|---|---|---|---|---|---|---|
| (A) | Sophorolipid A2 | 26.20 | 20.00 | 14.00 | 8.80 | 35.00 | |
| (B) | Potassium internal olefin sulfonate B3 | 8.80 | 15.00 | 21.00 | 26.20 | | 35.00 |
| (C) | Ion exchange water | Balance | Balance | Balance | Balance | Balance | Balance |
| | Total | 100.00 | 100.00 | 100.00 | 100.00 | 100.00 | 100.00 |
| | (A) + (B) | 35.00 | 35.00 | 35.00 | 35.00 | 35.00 | 35.00 |
| | (A)/(B) | 3.00 | 1.33 | 0.67 | 0.33 | - | - |
| | (A)/{(A) + (B)} | 0.75 | 0.57 | 0.40 | 0.25 | 1.00 | 0.00 |
| Cleansing solution | pH at 25°C | 8.5 | 8.5 | 8.5 | 8.5 | 8.5 | 8.5 |
| | Cleansing performance of cleansing solution I (%) | 34.4 | 22.4 | 29.1 | 27.5 | 24.5 | 27.9 |
| | Cleansing performance of cleansing solution II (%) | 9.0 | 12.7 | 10.7 | 10.0 | 8.1 | 7.9 |

[Examples 3-1 to 3-3 and Comparative Example 3-1]

Each cleansing composition was prepared in the same manner as in Example 1-1 in accordance with the compositional profile shown in Table 5.
Subsequently, the obtained cleansing composition was used in measurement and evaluation for the cleansing composition in the same manner as in Example 1-1.
Subsequently, 2.6 mL of the obtained cleansing composition was collected, and a cleansing solution was prepared in the same manner as in Example 1-1.
Subsequently, the obtained cleansing solution was used in measurement and evaluation for the cleansing solution (temperature: 25°C) in the same manner as in Example 1-1.
The results are shown in Table 5.

[Table 5]

| | | | Example 3-1 | Example 3-2 | Example 3-3 | Comparative Example 3-1 |
|---|---|---|---|---|---|---|
| (A) | Sophorolipid A1 | | 26.20 | 20.00 | 8.80 | 35.00 |
| (B) | Potassium internal olefin sulfonate B3 | | 8.80 | 15.00 | 26.20 | |
| (C) | Ion exchange water | | Balance | Balance | Balance | Balance |
| | Total | | 100.00 | 100.00 | 100.00 | 100.00 |
| | (A) + (B) | | 35.00 | 35.00 | 35.00 | 35.00 |
| | (A)/(B) | | 2.98 | 1.33 | 0.33 | - |
| | (A)/{(A) + (B)} | | 0.75 | 0.57 | 0.25 | 1.00 |
| Cleansing composition | | pH at 25°C | 4.6 | 4.2 | 3.6 | 3.4 |
| | | Fluidity at 25°C | X | X | X | Z |
| | | Colorless transparency at 25°C | C | C | A | E |
| Cleansing solution | | pH at 25°C | 8.5 | 8.5 | 8.5 | 8.5 |
| | | Cleansing performance at temperature of 25°C (%) | 34.0 | 27.2 | 23.5 | 27.0 |

[Examples 4-1 to 4-4 and Comparative Examples 4-1 and 4-2]

Each cleansing composition was prepared in the same manner as in Example 1-1 in accordance with the compositional profile shown in Table 6.

Subsequently, the obtained cleansing composition was used in measurement and evaluation for the cleansing composition in the same manner as in Example 1-1.

Subsequently, 21 mL of the obtained cleansing composition was collected and mixed with 2.6 mL of the model 4 000 °DH water and ion exchange water such that the amount of a liquid was 700 mL to prepare a cleansing solution having a total content of the component (A) and the component (B) of 0.15% by mass and a hardness of 15 °DH. The pH of the cleansing solution was appropriately adjusted to the value shown in Table 6 using 1.0 mol/L hydrochloric acid and a 0.5 mol/L aqueous potassium hydroxide solution.

<<Evaluation of cleansing performance of cleansing solution for contaminated glass or resin to be cleansed>>

1-1.) Preparation of model artificial sebum-contaminated glass

200 mL of the model artificial sebum liquid used in the preparation of the model artificial sebum-contaminated cloth in Example 1-1 was mixed with 600 mL of chloroform (manufactured by FUJIFILM Wako Pure Chemical Corp.), and the mixture was stirred until uniform using a stirrer at 25°C. Slide glass (Super Frost Slide Glass S2441, manufactured by Matsunami Glass Ind., Ltd.) was weighed, then dipped in the prepared artificial sebum solution, and pulled out thereof so that the model fat/oil was attached to the slide glass. Subsequently, the slide glass was left standing at 25°C for 2 hours so that the model fat/oil was solidified on the slide glass to prepare model artificial sebum-contaminated glass. The glass was weighed as glass after artificial sebum contamination.

1-2.) Preparation of model artificial sebum-contaminated resin

A model artificial sebum-contaminated resin was prepared in the same manner as in the procedure 1-1.) except that the slide glass was changed to a polypropylene test piece (PP-N-AN, 1.0 mm × 25 mm × 70 mm, manufactured by Standard Test Piece Co., Ltd.). The resin was weighed as a resin after artificial sebum contamination.

2.) Evaluation of cleansing performance

Four pieces of the model artificial sebum-contaminated glass prepared in the procedure 1-1.) or four pieces of the model artificial sebum-contaminated resin prepared in the procedure 1-2.) were loaded in a Leenerts tester. The cleansing solution was placed in a 1 L beaker, and the liquid temperature was adjusted to 30°C. The Leenerts tester with the sample

loaded therein was gently dipped in the cleansing solution and rapidly stirred at 250 rpm for 3 minutes. Subsequently, the Leenerts tester was pulled out thereof, dipped in 700 mL of ion exchange water, and stirred at 250 rpm for 1 minute. The test pieces thus stirred were dried overnight at room temperature, and the slide glass was weighed. A cleansing rate (%) was calculated according to the following expression (y), and an average value of the four pieces each of the glass was determined and used as an index of evaluation.

$$\text{Cleansing rate (\%)} \approx (Rw - Rs) / (Rw - R0) \times 100 \ldots \quad (y)$$

Rw: Mass (g) of the glass or the resin after artificial sebum contamination
Rs: Mass (g) of the glass or the resin after cleansing
R0: Mass (g) of the glass or the resin before artificial sebum contamination

[Table 6]

| | | Example 4-1 | Example 4-2 | Example 4-3 | Example 4-4 | Comparative Example 4-1 | Comparative Example 4-2 |
|---|---|---|---|---|---|---|---|
| (A) | Sophorolipid A2 | 3.75 | 2.50 | 2.00 | 1.25 | 5.00 | |
| (B) | Potassium internal olefin sulfonate B3 | 1.25 | 2.50 | 3.00 | 3.75 | | 5.00 |
| | 1 mol/L hydrochloric acid*4 | q.s. | q.s. | q.s. | q.s. | q.s. | q.s. |
| (C) | Ion exchange water | Balance | Balance | Balance | Balance | Balance | Balance |
| | Total | 100.00 | 100.00 | 100.00 | 100.00 | 100.00 | 100.00 |
| | (A) + (B) | 5.00 | 5.00 | 5.00 | 5.00 | 5.00 | 5.00 |
| | (A)/(B) | 3.00 | 1.00 | 0.67 | 0.33 | - | - |
| | (A)/{(A) + (B)} | 0.75 | 0.50 | 0.40 | 0.25 | 1.00 | 0.00 |
| Cleansing composition | pH at 25°C | 8.5 | 8.5 | 8.5 | 8.5 | 8.5 | 8.5 |
| | Colorless transparency at 25°C | C | B | B | A | D | A |
| Cleansing solution | pH at 25°C | 8.5 | 8.5 | 8.5 | 8.5 | 8.5 | 8.5 |
| | Cleasing performance for artificial sebum-contaminated resin (%) | 59.0 | 47.7 | 44.0 | 38.2 | 26.3 | 43.6 |
| | Cleasing performance for artificial sebum-contaminated glass (%) | 40.6 | 40.7 | 42.1 | 39.1 | 22.0 | 36.3 |
| *4: 1 mol/L hydrochloric acid, manufactured by FUJIFILM Wako Pure Chemical Corp. | | | | | | | |

[Examples 5-1 to 5-3 and Comparative Example 5-1]

Each cleansing composition was prepared in the same manner as in Example 1-1 in accordance with the compositional profile shown in Table 7.

Subsequently, the obtained cleansing composition was used in measurement for the cleansing composition in the same manner as in Example 1-1.

Subsequently, 3 mL of the obtained cleansing composition was collected, and a cleansing solution was subjected to measurement and evaluation for the cleansing solution (temperature: 30°C) in the same manner as in Example 4-1.
The results are shown in Table 7.

[Table 7]

| | | | Example 5-1 | Example 5-2 | Example 5-3 | Comparative Example 5-1 |
|---|---|---|---|---|---|---|
| (A) | Sophorolipid A1 | | 3.75 | 2.50 | 1.25 | 5.00 |
| (B) | Potassium internal olefin sulfonate B3 | | 1.25 | 2.50 | 3.75 | |
| | 0.5 mol/L aqueous potassium hydroxide solution*5 | | q.s. | q.s. | q.s. | q.s. |
| (C) | Ion exchange water | | Balance | Balance | Balance | Balance |
| | Total | | 100.00 | 100.00 | 100.00 | 100.00 |
| | (A) + (B) | | 5.00 | 5.00 | 5.00 | 5.00 |
| | (A)/(B) | | 3.00 | 1.00 | 0.33 | - |
| | (A)/{(A) + (B)} | | 0.75 | 0.50 | 0.25 | 1.00 |
| Cleansing composition | pH at 25°C | | 8.5 | 8.5 | 8.5 | 8.5 |
| | Colorless transparency at 25°C | | C | B | A | E |
| Cleansing solution | pH at 25°C | | 8.5 | 8.5 | 8.5 | 8.5 |
| | Cleansing performance for artificial sebum-contaminated glass (%) | | 39.5 | 37.7 | 42.3 | 32.8 |
| *5: 0.5 mol/L aqueous potassium hydroxide solution, manufactured by FUJIFILM Wako Pure Chemical Corp. | | | | | | |

[Examples 6-1 to 6-8 and Comparative Examples 6-1 to 6-3]

Each cleansing composition was prepared in the same manner as in Example 1-1 in accordance with the compositional profile shown in Table 8.
Subsequently, the obtained cleansing composition was used in measurement and evaluation for the cleansing composition in the same manner as in Example 1-1.
Subsequently, 2.25 mL of the obtained cleansing composition was collected, and a cleansing solution (total content of the component (A) and the component (B): 0.15% by mass, hardness: 15 °DH) was prepared in the same manner as in Example 1-1.
Subsequently, the obtained cleansing solution was used in measurement and evaluation for the cleansing solution (temperature: 25°C) in the same manner as in Example 1-1.
The results are shown in Table 8.

[Table 8]

| | | Example 6-1 | Example 6-2 | Example 6-3 | Example 6-4 | Example 6-5 | Example 6-6 | Example 6-7 | Example 6-8 | Comparative Example 6-1 | Comparative Example 6-2 | Comparative Example 6-3 |
|---|---|---|---|---|---|---|---|---|---|---|---|---|
| (A) | Sophorolipid A1 | 30.00 | 22.86 | 16.00 | 10.00 | | | | | | 40.00 | |
| | Sophorolipid A3 | | | | | 30.00 | 22.86 | 16.00 | 10.00 | | | 40.00 |
| (B) | Sodium internal olefin sulfonate B1 | 10.00 | 17.14 | 24.00 | 30.00 | 10.00 | 17.14 | 24.00 | 30.00 | 40.00 | | |
| (C) | Ion exchange water | Balance | Balance | Balance | Balance | Balance | Balance | Balance | Balance | Balance | Balance | Balance |
| | Total | 100.00 | 100.00 | 100.00 | 100.00 | 100.00 | 100.00 | 100.00 | 100.00 | 100.00 | 100.00 | 100.00 |
| | (A) + (B) (or (A') + (B)) | 40.00 | 40.00 | 40.00 | 40.00 | 40.00 | 40.00 | 40.00 | 40.00 | 40.00 | 40.00 | 40.00 |
| | (A)/(B) (or (A')/(B)) | 3.00 | 1.33 | 0.67 | 0.33 | 3.00 | 1.33 | 0.67 | 0.33 | - | 1.00 | 1.00 |
| | (A)/{(A) + (B)} (or (A')/{(A') + (B)}) | 0.75 | 0.57 | 0.40 | 0.25 | 0.75 | 0.57 | 0.40 | 0.25 | 0.00 | 1.00 | 1.00 |
| Cleansing composition | pH at 25°C | 4.4 | 4.9 | 5.7 | 6.6 | 6.3 | 6.7 | 7.0 | 7.7 | 8.1 | 6.1 | 6.1 |
| | Fluidity at 25°C | X | X | X | X | X | X | X | X | Z | Z | Z |
| | Colorless transparency at 25°C | C | C | B | A | C | C | B | A | A | D | D |
| Cleansing solution | pH at 25°C | 8.5 | 8.5 | 8.5 | 8.5 | 8.5 | 8.5 | 8.5 | 8.5 | 8.5 | 8.5 | 8.5 |
| | Cleansing performance at temperature of 25°C (%) | 32.9 | 30.6 | 28.6 | 28.5 | 32.2 | 28.1 | 26.7 | 27.6 | 22.1 | 28.8 | 28.8 |

[Examples 7-1 to 7-4]

Each cleansing composition was prepared in the same manner as in Example 1-1 in accordance with the compositional profile shown in Table 9.

Subsequently, the obtained cleansing composition was used in measurement and evaluation for the cleansing composition in the same manner as in Example 1-1.

Subsequently, 2.25 mL of the obtained cleansing composition was collected, and a cleansing solution (total content of the component (A) and the component (B): 0.15% by mass, hardness: 15 °DH) was prepared in the same manner as in Example 1-1.

Subsequently, the obtained cleansing solution was used in measurement and evaluation for the cleansing solution (temperature: 25°C) in the same manner as in Example 1-1.

The results are shown in Table 9.

[Table 9]

| | | | Example 7-1 | Example 7-2 | Example 7-3 | Example 7-4 |
|---|---|---|---|---|---|---|
| (A) | Sophorolipid A3 | | 26.25 | 20.00 | 14.00 | 8.75 |
| (B) | Potassium internal olefin sulfonate B3 | | 8.75 | 15.00 | 21.00 | 26.25 |
| (C) | Ion exchange water | | Balance | Balance | Balance | Balance |
| | Total | | 100.00 | 100.00 | 100.00 | 100.00 |
| | (A) + (B) (or (A') + (B)) | | 35.00 | 35.00 | 35.00 | 35.00 |
| | (A)/(B) (or (A')/(B)) | | 3.00 | 1.33 | 0.67 | 0.33 |
| | (A)/{(A) + (B)} (or (A')/{(A') + (B)}) | | 0.75 | 0.57 | 0.40 | 0.25 |
| Cleansing composition | | pH at 25°C | 6.5 | 6.6 | 6.7 | 6.8 |
| | | Fluidity at 25°C | X | X | X | X |
| | | Colorless transparency at 25°C | C | C | B | A |
| Cleansing solution | pH at 25°C | 8.5 | 8.5 | 8.5 | 8.5 | |
| | Cleansing performance at temperature of 25°C (%) | 33.7 | 31.2 | 26.7 | 29.4 | |

[Examples 8-1 to 8-3 and Comparative Example 8-1]

The component (A), the component (B), and the component (C) were weighed into a glass beaker in accordance with the compositional profile shown in Table 10, and further, pH was appropriately adjusted to 5 using 1.0 mol/L hydrochloric acid and a 0.5 mol/L aqueous potassium hydroxide solution to prepare a cleansing composition.

<<Evaluation of protein elution suppressing effect>>

0.2 g of zein (manufactured by FUJIFILM Wako Pure Chemical Corp.) was weighed into a centrifugal tube (manufactured by AGC Techno Glass Co., Ltd.) with a measured weight. Further, 10 mL of the cleansing composition was placed therein, and the mixture was vigorously stirred for 5 seconds. Subsequently, the resultant was shaken at 110 rpm for 2 hours using a shaker temperature-adjusted to 32°C. After shaking, a supernatant was removed by centrifugation at 3 000 rpm for 90 minutes, and the residue was then resuspended by the addition of 10 mL of ion exchange water and centrifuged in the same manner as above.

The above step of adding ion exchange water for resuspension, followed by centrifugation was repeated five times, and the surfactant remaining in precipitates was washed. The precipitates thus washed were placed in a dryer temperature-adjusted to 80°C, and dried for 2 days or longer, and a weight after drying was measured.

Based on the obtained measurement value, the amount of zein eluted per 100 mL of the cleansing composition (g-zein/100 mL solution) was calculated before and after the test. Based on the obtained value of the amount of zein eluted, a protein elution suppressing effect was evaluated in accordance with the following criteria.

A: The amount of zein eluted was less than 1 g-zein/100 mL solution, which indicates a high protein elution suppressing effect and causes very small damage on proteins.

B: The amount of zein eluted was equal to or more than 1 g-zein/100 mL solution and less than 1.3 g-zein/100 mL solution, which indicates a slightly low protein elution suppressing effect and causes slightly small damage on proteins.

C: The amount of zein eluted was equal to or more than 1.3 g-zein/100 mL solution, which indicates a low protein elution suppressing effect and causes large damage on proteins.

The results are shown in Table 10.

[Table 10]

| | | Example 8-1 | Example 8-2 | Example 8-3 | Compara tive Example 8-1 |
|---|---|---|---|---|---|
| (A) | Sophorolipid A3 | 0.50 | 0.40 | 0.25 | |
| (B) | Potassium internal olefin sulfonate B3 | 0.50 | 0.60 | 0.75 | 1.00 |
| | 0.5 mol/L aqueous potassium hydroxide solution*5 | q.s. | q.s. | q.s. | q.s. |
| (C) | Ion exchange water | Balance | Balance | Balance | Balance |
| | Total | 100.00 | 100.00 | 100.00 | 100.00 |
| | (A) + (B) | 1.00 | 1.00 | 1.00 | 1.00 |
| | (A)/(B) | 1.00 | 0.67 | 0.33 | - |
| | (A)/{(A) + (B)} | 0.50 | 0.40 | 0.25 | 0.00 |
| Cleansing composition | pH at 25°C | 7.0 | 7.0 | 7.0 | 7.0 |
| | Amount of zein eluted (g-zein/ 100 mL solution) | 0.5 | 0.8 | 1.2 | 1.4 |
| | Protein elution suppressing effect | A | A | B | C |
| *5: Same as in Table 7 | | | | | |

[Examples 9-1 to 9-7 and Comparative Example 9-1]

Each cleansing composition was prepared in the same manner as in Example 8-1 in accordance with the compositional profile shown in Table 11.

Subsequently, the obtained cleansing composition was used in evaluation for the cleansing composition in the same manner as in Example 8-1.

The results are shown in Table 11.

[Table 11]

| | | Example 9-1 | Example 9-2 | Example 9-3 | Example 9-4 | Example 9-5 | Example 9-6 | Example 9-7 | Comparative Example 9-1 |
|---|---|---|---|---|---|---|---|---|---|
| (A) | Sophorolipid A1 | 0.25 | 0.25 | | | | | | |
| | Sophorolipid A2 | | | 0.25 | 0.25 | | | | |
| | Sophorolipid A3 | | | | | 0.25 | 0.25 | 0.25 | |
| (B) | Potassium internal olefin sulfonate B3 | 0.75 | 0.75 | 0.75 | 0.75 | 0.75 | 0.75 | 0.75 | 1.00 |
| | 0.5 mol/L aqueous potassium hydroxide solution*5 | q.s. | q.s. | q.s. | q.s. | q.s. | q.s. | q.s. | q.s. |
| (C) | Ion exchange water | Balance | Balance | Balance | Balance | Balance | Balance | Balance | Balance |
| | Total | 100.00 | 100.00 | 100.00 | 100.00 | 100.00 | 100.00 | 100.00 | 100.00 |
| | (A) + (B) | 1.00 | 1.00 | 1.00 | 1.00 | 1.00 | 1.00 | 1.00 | 1.00 |
| | (A)/(B) | 0.33 | 0.33 | 0.33 | 0.33 | 0.33 | 0.33 | 0.33 | - |
| | (A)/{(A) + (B)} | 0.25 | 0.25 | 0.25 | 0.25 | 0.25 | 0.25 | 0.25 | 0.00 |
| Cleansing composition | pH at 25°C | 5.0 | 3.0 | 5.0 | 3.0 | 7.0 | 5.0 | 3.0 | 5.0 |
| | Amount of zein eluted (g-zein/ 100 mL solution) | 0.7 | 0.4 | 1.0 | 0.7 | 1.0 | 0.7 | 0.5 | 1.3 |
| | Protein elution suppressing effect | A | A | B | A | B | A | A | C |

*5: Same as in Table 7

[Example 10-1 and Comparative Example 10-1]

Each cleansing composition was prepared in the same manner as in Example 8-1 in accordance with the compositional profile shown in Table 12.

Subsequently, 2.0 mL of the obtained cleansing composition was collected and mixed with 0.01 mL of the model 4 000 °DH water and ion exchange water such that the amount of a liquid was 10 mL to prepare a cleansing solution (total content of the component (A) and the component (B): 1% by mass, hardness: 4 °DH) .

Subsequently, the obtained cleansing solution was used in each measurement and evaluation for the cleansing solution in the same manner as in Example 8-1.

The results are shown in Table 12.

[Table 12]

| | | Example 10-1 | Comparative Example 10-1 |
|---|---|---|---|
| (A) | Sophorolipid A1 | 1.25 | |
| (B) | Potassium internal olefin sulfonate B3 | 3.75 | 5.00 |
| | 0.5 mol/L aqueous potassium hydroxide solution*5 | q.s. | q.s. |
| (C) | Ion exchange water | Balance | Balance |
| | Total | 100.00 | 100.00 |
| | (A) + (B) | 5.00 | 5.00 |
| | (A)/(B) | 0.33 | - |
| | (A)/{(A) + (B)} | 0.25 | 0.00 |
| Cleansing composition | pH at 25°C | 5.0 | 5.0 |
| Cleansing solution | pH at 25°C | 5.0 | 5.0 |
| | Amount of zein eluted (g-zein/ 100 mL solution) | 0.5 | 1.3 |
| | Protein elution suppressing effect | A | C |
| *5: Same as in Table 7 | | | |

[Examples 11-1 to 11-5 and Comparative Examples 11-1 and 11-2]

Each cleansing composition was prepared in the same manner as in Example 8-1 in accordance with the compositional profile shown in Table 13.

Subsequently, the obtained cleansing composition was used in evaluation for the cleansing composition in the same manner as in Example 8-1.

The results are shown in Table 13.

[Table 13]

| | | Example 11-1 | Example 11-2 | Example 11-3 | Example 11-4 | Example 11-5 | Comparative Example 11-1 | Comparative Example 11-2 |
|---|---|---|---|---|---|---|---|---|
| (A) | Sophorolipid A1 | | | | 0.25 | | | |
| | Sophorolipid A3 | 0.50 | 0.40 | 0.25 | | 0.25 | | |
| (B) | Sodium internal olefin sulfonate B1 | 0.50 | 0.60 | 0.75 | | | 1.00 | |
| | Sodium internal olefin sulfonate B2 | | | | 0.75 | 0.75 | | 1.00 |

(continued)

| | | Example 11-1 | Example 11-2 | Example 11-3 | Example 11-4 | Example 11-5 | Comparative Example 11-1 | Comparative Example 11-2 |
|---|---|---|---|---|---|---|---|---|
| | 0.5 mol/L aqueous potassium hydroxide solution*5 | q.s. | q.s. | q.s. | q.s. | q.s. | q.s. | q.s. |
| (C) | Ion exchange water | Balance | Balance | Balance | Balance | Balance | Balance | Balance |
| | Total | 100.00 | 100.00 | 100.00 | 100.00 | 100.00 | 100.00 | 100.00 |
| | (A) + (B) | 1.00 | 1.00 | 1.00 | 1.00 | 1.00 | 1.00 | 1.00 |
| | (A)/(B) | 1.00 | 0.67 | 0.33 | 0.33 | 0.33 | - | - |
| | (A)/{(A) + (B)} | 0.50 | 0.40 | 0.25 | 0.25 | 0.25 | 0.00 | 0.00 |
| Cleansing composition | pH at 25°C | 5.0 | 5.0 | 5.0 | 5.0 | 5.0 | 5.0 | 5.0 |
| | Amount of zein eluted (g-zein/ 100 mL solution) | 0.3 | 0.6 | 1.1 | 0.9 | 1.0 | 1.8 | 1.6 |
| | Protein elution suppressing effect | A | A | B | A | B | C | C |
| *5: Same as in Table 7 | | | | | | | | |

[Examples 12-1 and 12-2 and Comparative Examples 12-1 and 12-2]

Each cleansing composition was prepared in the same manner as in Example 8-1 in accordance with the compositional profile shown in Table 14.

<<Evaluation of cleansing performance of cleansing solution for the skin to be cleansed>>

2% by mass of carbon black was dispersed in 98% by mass of the model sebum for coloring, and melted. 8 μL of the resultant was applied with a size of 3 cm in diameter to an inner forearm site. The solution thus applied was left for 30 minutes and then coagulated, and 8 mg of the cleansing solution was applied thereto, massaged 20 times with a forefinger in a circular motion, and rinsed with ion exchange water for 30 seconds. Subsequently, lightness (L value) was determined before and after cleansing using a chromameter CR-300 (manufactured by Minolta Co., Ltd.), and a cleansing rate was calculated according to the following expression (z) and used as an index of evaluation.

$$\text{Cleansing rate (\%)} \approx (Lw - Ls) / (Lw - L0) \times 100 \quad \ldots (z)$$

Lw: L value after artificial sebum contamination
Ls: L value after cleansing
L0: L value before artificial sebum contamination

The results are shown in Table 14.

[Table 14]

| | | Example 12-1 | Example 12-2 | Comparative Example 12-1 | Comparative Example 12-2 |
|---|---|---|---|---|---|
| (A) | Sophorolipid A3 | 0.50 | 0.40 | | 1.00 |
| (B) | Sodium internal olefin sulfonate B1 | 0.50 | 0.60 | 1.00 | |
| | 0.5 mol/L aqueous potassium hydroxide solution*5 | q.s. | q.s. | q.s. | q.s. |
| (C) | Ion exchange water | Balance | Balance | Balance | Balance |
| | Total | 100.00 | 100.00 | 100.00 | 100.00 |
| | (A) + (B) | 1.00 | 1.00 | 1.00 | 1.00 |
| | (A)/(B) | 1.00 | 0.67 | - | - |
| | (A)/{(A) + (B)} | 0.50 | 0.40 | 0.00 | 1.00 |
| Cleansing composition | pH at 25°C | 5.0 | 5.0 | 5.0 | 5.0 |
| | Cleansing performance for sebum on skin (%) | 76.7 | 67.3 | 66.6 | 45.4 |
| *5: Same as in Table 7 | | | | | |

Hereinafter, Formulation Examples will be shown as to the embodiments of the present invention. The content of each component is indicated by % by mass unless otherwise specified in tables.

Formulation Examples 1-1 to 1-16 shown in Table 15 each provide a cleansing composition for apparel. Its pH at 25°C is appropriately adjusted to 8.5 using potassium hydroxide or citric acid, and the whole amount is set to 100% by mass.

Formulation Examples 2-1 to 2-15 shown in Table 16 each provide a cleansing composition for hard surface. Its pH at 25°C is appropriately adjusted to 9.5 using potassium hydroxide or citric acid, and the whole amount is set to 100% by mass.

Formulation Examples 3-1 to 3-8 shown in Table 17 each provide a cleansing composition for the skin. Its pH at 25°C is appropriately adjusted to 5.5 using potassium hydroxide or lactic acid, and the whole amount is set to 100% by mass.

In all of these Formulation Examples, the same effects as in Examples described above can be obtained.

[Table 15]

| | Formulation Example 1-1 | Formulation Example 1-2 | Formulation Example 1-3 | Formulation Example 1-4 | Formulation Example 1-5 | Formulation Example 1-6 | Formulation Example 1-7 | Formulation Example 1-8 | Formulation Example 1-9 | Formulation Example 1-10 | Formulation Example 1-11 | Formulation Example 1-12 | Formulation Example 1-13 | Formulation Example 1-14 | Formulation Example 1-15 | Formulation Example 1-16 |
|---|---|---|---|---|---|---|---|---|---|---|---|---|---|---|---|---|
| Sophorolipid A1 | 8.8 | - | - | - | - | - | 8.8 | - | - | - | - | - | - | - | - | - |
| Sophorolipid A2 | - | 14.0 | 14.0 | 14.0 | 14.0 | 17.5 | - | 14.0 | - | 14.0 | - | 14.0 | - | 14.0 | - | - |
| Sophorolipid A3 | - | - | - | - | - | - | - | - | 14.0 | - | 14.0 | - | 14.0 | - | 14.0 | 14.0 |
| Sodium internal olefin sulfonate B1 | 26.2 | 21.0 | - | 21.0 | 21.0 | 17.5 | - | - | - | - | - | - | - | - | - | - |
| Sodium internal olefin sulfonate B2 | - | - | 21.0 | - | - | - | - | - | - | - | - | - | - | - | - | - |
| Potassium internal olefin sulfonate B3 | - | - | - | - | - | - | 26.2 | 21.0 | 21.0 | - | - | 21.0 | 21.0 | 21.0 | 21.0 | - |
| Potassium internal olefin sulfonate B4 | - | - | - | - | - | | - | - | - | 21.0 | 21.0 | - | - | - | - | 21.0 |
| Propylene glycol | - | - | - | 3 | 3 | 3 | - | - | - | - | - | 3 | 3 | 3 | 3 | 3 |
| Ethanol | - | - | - | - | 3 | 3 | - | - | - | - | - | - | - | 3 | 3 | 3 |
| α-Amylase | - | - | - | - | 0.3 | 0.1 | - | - | - | - | - | - | - | 0.3 | 0.3 | 0.3 |
| Lipase | - | - | - | - | 0.1 | 0.1 | - | - | - | - | - | - | - | 0.1 | 0.1 | 0.1 |
| Cellulase | - | - | - | - | 0.1 | 0.1 | - | - | - | - | - | - | - | 0.1 | 0.1 | 0.1 |
| Mannase | - | - | - | - | 0.1 | 0.1 | - | - | - | - | - | - | - | 0.1 | 0.1 | 0.1 |
| Polyacrylic acid | - | - | - | - | 0.15 | 0.15 | - | - | - | - | - | - | - | 0.15 | 0.15 | 0.15 |
| Preservative | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 |
| Fragrance | - | - | - | 0.3 | 0.3 | 0.3 | - | - | - | - | - | 0.3 | 0.3 | 0.3 | 0.3 | 0.3 |
| Potassium hydroxide/citric acid | q.s. | | | | | | | | | | | | | | | |
| Ion exchange water | Balance | | | | | | | | | | | | | | | |
| pH at 25°C | 8.5 | | | | | | | | | | | | | | | |

[Table 16]

| | Formulation Example 2-1 | Formulation Example 2-2 | Formulation Example 2-3 | Formulation Example 2-4 | Formulation Example 2-5 | Formulation Example 2-6 | Formulation Example 2-7 | Formulation Example 2-8 | Formulation Example 2-9 | Formulation Example 2-10 | Formulation Example 2-11 | Formulation Example 2-12 | Formulation Example 2-13 | Formulation Example 2-14 | Formulation Example 2-15 |
|---|---|---|---|---|---|---|---|---|---|---|---|---|---|---|---|
| Sophorolipid A1 | 0.2 | 0.1 | 1.0 | 4.0 | - | 0.2 | 0.2 | 0.2 | 0.2 | - | - | - | - | - | 1.0 |
| Sophorolipid A2 | - | - | - | - | 0.2 | - | - | - | - | - | - | - | - | 1.0 | - |
| Sophorolipid A3 | - | - | - | - | - | - | - | - | - | 0.2 | 1.0 | 0.2 | 1 | - | - |
| Sodium internal olefin sulfonate B1 | 0.3 | 0.2 | 1.5 | 6.0 | 0.3 | - | 0.3 | 0.3 | 0.3 | - | - | - | - | - | - |
| Sodium internal olefin sulfonate B2 | - | - | - | - | - | 0.3 | - | - | - | - | - | - | - | - | - |
| Potassium internal olefin sulfonate B3 | - | - | - | - | - | - | - | - | - | 0.3 | 1.5 | - | - | 1.5 | 1.5 |
| Potassium internal olefin sulfonate B4 | - | - | - | - | - | - | - | - | - | - | - | 0.3 | 1.5 | - | - |
| Propylene glycol | - | - | - | - | - | - | 2 | 2 | 2 | - | - | - | - | - | - |
| Ethanol | - | - | - | - | - | - | - | - | 4 | - | - | - | - | - | - |
| Preservative | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 |
| Fragrance | 0.1 | 0.1 | 0.1 | 0.1 | 0.2 | 0.2 | 0.3 | 0.3 | 0.3 | 0.1 | 0.1 | 0.1 | 0.1 | 0.1 | 0.1 |
| Potassium hydroxide/citric acid | q.s. | | | | | | | | | | | | | | |
| Ion exchange water | Balance | | | | | | | | | | | | | | |
| pH at 25°C | 9.5 | | | | | | | | | | | | | | |

[Table 17]

| | Formulation Example 3-1 | Formulation Example 3-2 | Formulation Example 3-3 | Formulation Example 3-4 | Formulation Example 3-5 | Formulation Example 3-6 | Formulation Example 3-7 | Formulation Example 3-8 |
|---|---|---|---|---|---|---|---|---|
| Sophorolipid A1 | 2.5 | - | 3.0 | 3.0 | 3.0 | 3.0 | - | - |
| Sophorolipid A2 | - | 3.0 | - | - | - | - | - | - |
| Sophorolipid A3 | - | - | - | - | - | - | 2.5 | 3 |
| Sodium internal olefin sulfonate B1 | 7.5 | 7.0 | - | 7.0 | 7.0 | 7.0 | 7.5 | 7.0 |
| Sodium internal olefin sulfonate B2 | - | - | 7.0 | - | - | - | - | - |
| Propylene glycol | - | - | - | 2 | 2 | 2 | - | - |
| Ethanol | - | - | - | - | - | 4 | - | - |
| Ethylenediaminetetraacetic acid | - | - | - | - | - | 0.15 | - | - |
| Preservative | 0.1 | 0.1 | 0.1 | 0.1 | 0.1 | 0.1 | 0.1 | 0.1 |
| Fragrance | 0.1 | 0.2 | 0.2 | 0.3 | 0.3 | 0.3 | 0.1 | 0.1 |
| Potassium hvdroxide/lactic acid | q.s. | | | | | | | |
| Ion exchange water | Balance | | | | | | | |
| pH at 25°C | 5.5 | | | | | | | |

## Claims

1. A cleansing composition comprising the following components (A) to (C):

   (A) a glycolipid biosurfactant,
   (B) an internal olefin sulfonate having 8 or more and 24 or less carbon atoms, wherein a content of an internal olefin sulfonate having a sulfonic acid group at the 2-position is 40% by mass or less, and
   (C) water.

2. The cleansing composition according to claim 1, which is for cleansing a hard-surface article, an apparel product, or a body.

3. The cleansing composition according to claim 1 or 2, wherein a mass ratio of a content of the component (A) to a content of the component (B) ((A)/(B)) is 0.0050 or more and 95.00 or less.

4. The cleansing composition according to claim 1 or 2, wherein a mass ratio of a content of an internal olefin sulfonate (IO-1S) having 8 or more and 24 or less carbon atoms and having a sulfonic acid group at the 2- or higher and 4- or lower position to a content of an internal olefin sulfonate (IO-2S) having 8 or more and 24 or less carbon atoms and having a sulfonic acid group at the 5- or higher position ((IO-1S)/(IO-2S)), in the component (B) is 0.50 or more and 6.5 or less.

5. The cleansing composition according to claim 1 or 2, wherein a mass ratio of a content of the component (A) to a total content of the component (A) and the component (B) ((A)/{(A) + (B)}) is 0.0050 or more and 0.99 or less.

6. The cleansing composition according to claim 1 or 2, wherein the component (A) is one or more selected from the group consisting of sophorolipid, rhamnolipid, trehalose lipid, and mannosyl alditol lipid.

7. The cleansing composition according to claim 1 or 2, wherein a total content of the component (A) and the component (B) is 0.15% by mass or more and 55% by mass or less.

8. The cleansing composition according to claim 1 or 2 for cleansing a hard-surface article or a body, wherein a total content of the component (A) and the component (B) is 0.15% by mass or more and 25% by mass or less.

9. The cleansing composition according to claim 1 or 2 for cleansing an apparel product, wherein a total content of the component (A) and the component (B) is 14% by mass or more and 55% by mass or less.

10. The cleansing composition according to claim 1 or 2, wherein a content of the component (C) is 30% by mass or more and 99% by mass or less.

11. A cleansing solution prepared by diluting the cleansing composition according to claim 1 or 2 1-fold or more and 10 000-fold or less with water.

12. A cleansing solution for cleansing a hard-surface article or a body, the cleansing solution being prepared by diluting the cleansing composition according to claim 1 or 2 1-fold or more and 100-fold or less with water.

13. A cleansing solution for cleansing an apparel product, the cleansing solution being prepared by diluting the cleansing composition according to claim 1 or 2 100-fold or more and 5 000-fold or less with water.

14. A cleansing method using the cleansing composition according to claim 1 or 2 and water, wherein a hardness of the water is 30 °DH or less.

15. The cleansing method according to claim 14, wherein a temperature of the water is 5°C or higher and 60°C or lower.

16. An aqueous solution of surfactant comprising the following components (A) to (C):

   (A) a glycolipid biosurfactant,
   (B) an internal olefin sulfonate having 8 or more and 24 or less carbon atoms, wherein a content of an internal olefin sulfonate having a sulfonic acid group at the 2-position is 40% by mass or less, and

(C) water.

**17.** A kit for cleansing, comprising:

an agent A comprising (A) a glycolipid biosurfactant; and
an agent B comprising (B) an internal olefin sulfonate having 8 or more and 24 or less carbon atoms, wherein a content of an internal olefin sulfonate having a sulfonic acid group at the 2-position is 40% by mass or less.

**INTERNATIONAL SEARCH REPORT**

International application No. **PCT/JP2024/038697**

**A. CLASSIFICATION OF SUBJECT MATTER**

***C11D 1/14***(2006.01)i; ***C11D 1/68***(2006.01)i; ***C11D 3/22***(2006.01)i
FI: C11D1/14; C11D3/22; C11D1/68

According to International Patent Classification (IPC) or to both national classification and IPC

**B. FIELDS SEARCHED**

Minimum documentation searched (classification system followed by classification symbols)

C11D; A61K; A61Q; B08B; C07C; C09K

Documentation searched other than minimum documentation to the extent that such documents are included in the fields searched

Published examined utility model applications of Japan 1922-1996
Published unexamined utility model applications of Japan 1971-2025
Registered utility model specifications of Japan 1996-2025
Published registered utility model applications of Japan 1994-2025

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2019-044187 A (KAO CORPORATION) 22 March 2019 (2019-03-22) | 1-17 |
| A | JP 2017-214571 A (KAO CORPORATION) 07 December 2017 (2017-12-07) | 1-17 |
| A | JP 2017-214576 A (KAO CORPORATION) 07 December 2017 (2017-12-07) | 1-17 |
| A | WO 2018/030328 A1 (KAO CORPORATION) 15 February 2018 (2018-02-15) | 1-17 |
| A | JP 6999211 B2 (SARAYA CO., LTD.) 18 January 2022 (2022-01-18) | 1-17 |
| A | US 2020/0199492 A1 (EVONIK DEGUSSA GMBH) 25 June 2020 (2020-06-25) | 1-17 |
| A | JP 5-59394 A (UNILEVER NV) 09 March 1993 (1993-03-09) | 1-17 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 January 2025** | **21 January 2025** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/038697**

| Patent document cited in search report | Publication date (day/month/year) | Patent family member(s) | Publication date (day/month/year) |
|---|---|---|---|
| JP 2019-044187 A | 22 March 2019 | US 2020/0190432 A1 | |
| | | WO 2019/049895 A1 | |
| | | EP 3680315 A1 | |
| | | CN 111032842 A | |
| JP 2017-214571 A | 07 December 2017 | US 2020/0318036 A1 | |
| | | WO 2017/209118 A1 | |
| | | EP 3467082 A1 | |
| | | CN 109219651 A | |
| JP 2017-214576 A | 07 December 2017 | US 2020/0231899 A1 | |
| | | WO 2017/209085 A1 | |
| | | EP 3467088 A1 | |
| | | CN 109312278 A | |
| WO 2018/030328 A1 | 15 February 2018 | US 2019/0169536 A1 | |
| | | EP 3498811 A1 | |
| | | CN 109563440 A | |
| JP 6999211 B2 | 18 January 2022 | US 2022/0257773 A1 | |
| | | WO 2020/250475 A1 | |
| | | EP 3984628 A1 | |
| | | CN 113966378 A | |
| US 2020/0199492 A1 | 25 June 2020 | WO 2018/197623 A1 | |
| | | EP 3615646 A1 | |
| | | CN 110719951 A | |
| JP 5-59394 A | 09 March 1993 | US 5417879 A | |
| | | EP 499434 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015091250 A
- US 20200199492


### Non-patent literature cited in the description


- *J. Am. Oil Chem. Soc.*, 1992, vol. 69, 39